# EUROPEAN PATENT SPECIFICATION

(11) **EP 2 564 830 B1**
(45) Date of publication and mention of the grant of the patent: **01.11.2017**
(21) Application number: 11774904.4
(22) Date of filing: 21.04.2011
(51) Int. Cl.: A61K 9/00, A61K 47/10, A61K 47/32, A61K 47/34, A61K 8/39, A61K 8/81, A61Q 19/00

(54) **AQUEOUS COMPOSITION CONTAINED IN CONTAINER**
IN EINEM BEHÄLTER AUFBEWAHRTE WÄSSRIGE ZUSAMMENSETZUNG
COMPOSITION AQUEUSE CONTENUE DANS UN CONTENANT

(30) Priority: 12.11.2010 JP 2010254185; 17.05.2010 JP 2010112859; 27.04.2010 JP 2010102160
(43) Date of publication of application: 06.03.2013
(73) Proprietor: Kao Corporation, Tokyo 103-8210 (JP)
(72) Inventor: MURATA, Takeshi, Odawara-shi Kanagawa 250-0002 (JP)
(74) Representative: Hoffmann Eitle
(86) International application number: PCT/JP2011/059818
(87) International publication number: WO 2011/136121

(56) References cited:
- EP-A1- 0 210 747
- WO-A2-03/090670
- WO-A2-2007/079253
- JP-A- 1 266 844
- JP-A- 2000 265 098
- JP-A- 2003 034 620
- JP-A- 2004 107 586
- JP-A- 2005 124 690
- JP-A- 2005 280 091
- JP-A- 2009 029 880
- US-A- 3 146 059

## Description

### [Field of the Invention]

The present invention relates to a cosmetic, drug or food contained in a container comprising a stable aqueous composition, with water evaporation from the aqueous phase inhibited.

### [Background of the Invention]

In the fields of cosmetics, drugs, quasi-drugs, foods, etc., many compositions containing water as a base are used. In such aqueous compositions, in order to prevent water in the compositions from evaporating, the compositions are stored in airtight containers in many cases. However, for example, opening of the lid in daily use causes gradual evaporation of water in an aqueous composition, which may change the physical properties or induce discoloration. In some containers, such as spray containers and pump containers, a part of discharged contents remains at the discharge opening and dries near the discharge opening. The contents adhering to the discharge opening cause changes in color and odor, resulting in deterioration in sense of use. In addition, the contents dry and solidify to cause clogging, resulting in impossible discharge in some cases.

In order to suppress solidification of contents and thereby to prevent clogging of a discharge container, proposed is blending a non-volatile liquid oil with one or more selected from materials that are solid at 25°C or less and/or coating-forming polymers at a specific ratio (see Patent Document 1). In order to suppress solidification of contents, however, a large amount of non-volatile oil is necessarily blended, which limits possible formulations. In addition, even if a specific amount of non-volatile oil is blended, the contents solidify in some cases, and clogging is not sufficiently prevented.

It is also proposed to improve long-term stability of an emulsion containing a high concentration of alcohol by blending polyoxyethylene behenyl ether having an average number of ethylene oxide added of 5 or more into the emulsion composition (see Patent Documents 2 and 3). Such a method, however, intends to prevent decreases in hardness and viscosity of the emulsion, and effects on water are not known at all.

Skin care cosmetics such as milky lotions, beauty essences, and creams are generally used as external skin preparations for providing moisture and flexibility to the skin. These compositions for external use on skin mainly contain polyols, water-holding water-soluble polymers, and oils. The polyol and the water-holding water-soluble polymer hold water on the skin for a long time by the water holding capacities of themselves; the oil forms a thin film on the skin to prevent water of the skin from evaporating into the air and to thereby keep water on the skin; and thereby the skin core cosmetics intend to show moisturizing effects.

In order to enhance the effects of a polyol and so on, a large amount of a polyol is necessary, and there is a problem that if does not so, the effects are low. However, a high content of a polyol tends to make the emulsion unstable and, thereby, tends to make ensuring stability difficult. As examples of using water-holding water-soluble polymers, cosmetics, which contains a water-soluble polymer such as a polymer or copolymer having 2-methacryloyloxyethyl phosphorylcholine as a structural monomer, and a water-holding polymer, and hydroxyalkyl cellulose, have been proposed and such cosmetics have an effect of highly occluding water (see Patent Documents 4 and 5). Increases in amounts thereof in order to obtain a sufficient moisturizing effect also have problems of causing an increase in sticky feeling or gelation or generating dregs that are aggregates of the polymer detached when being spread on the skin. Furthermore, even in a coating having a water holding capacity due to the water-soluble polymer, the effect of inhibiting water evaporation is insufficient. Thus, the effects are unsatisfactory.

In the case of blending oil, the occluding property of the oil is high in non-polar oils such as Vaseline and liquid paraffin, and the property decreases with an increase in polarity as in ester oil. Accordingly, compositions for external use on skin containing Vaseline are commercially available as those having high occluding effects.

However, the non-polar oil such as Vaseline or liquid paraffin generally has low compatibility with sebum and thereby moves on the skin even after application and causes a slimy feeling, and also the oil disadvantageously transfers to touched clothes or paper. In the case of cosmetics, such as foundation, where the oil is blended with powder, the powder easily transfers to clothes when the skin applied with such a cosmetic is brought into contact with the clothes.

### [Prior art list]

### [Patent Document]

[Patent Document 1] JP-A-2003-321321
[Patent Document 2] JP-A-1989-266844
[Patent Document 3] JP-A-1999-508253
[Patent Document 4] JP-A-2006-22047
[Patent Document 5] JP-A-2006-509718
[Patent Document 6] EP-A1-0210747
   Patent Document 6 discloses a multi-component, water-based, polymeric composition which comprises as evaporation-suppressing agent at least on surface-active, aliphatic compound having a carbon-carbon chain length of at least 16 atoms and at least one hydrophilic group, excluding aliphatic monocarboxylic acids. The evaporation suppressing agent may be a compound represented by the formula CH₃(CH₂)ₘ(OCH₂CHX)ₙ[O(CH₂)_{y}]_{z}OH, wherein m is 15 to 25, X is H or CH₃, n is 0 to 3, y is 2 to 4 and z is 0 to 3. The compositions may also comprise a film-forming resin such as alkyl acrylates, methacrylates, vinyl acetates.
[Patent Document 7] USP3146059
   Patent Document 7 is directed to organic compounds which suppress water-evaporation and are effective to inhibit loss of water, fall of water temperature and further drying of soil. For this purpose, a compound of the formula can be used: CH₃-(CH₂)ₙ-O-(CH₂-CH₂-O)ₙ-H, wherein m = 15 to 21 and n = 1 to 5. Such a compound may be combined with a water-soluble polymer, e.g. carboxymethyl cellulose or alginates, and water.
[Patent Document 8] WO-A2-2007/079253
   In Patent Document 8 compositions to retard the rate of water loss from a body of water, e.g. a swimming pool or lake, are described. The composition comprises a polyoxyethylene ether R-(OCH₂CH₂)ₙOH, wherein R is alkyl group with less than 16 carbon atoms and n = 1 to 5. The composition may further comprise a viscosity modifying agent such as carboxymethyl cellulose or dextranes.

### [Summary of the Invention]

The present invention provides a cosmetic, drug or food contained in a container comprising an aqueous composition, wherein the composition contains the following components (A), (B), and (C):
(A) a polyoxyethylene alkyl or alkenyl ether having an alkyl or alkenyl group having 20 to 24 carbon atoms and an average molar number of ethylene oxide added of 1.5 to 4,
(B) a water-soluble polymer, and
(C) water.

The present invention also provides a cosmetic, drug or food contained in a container, wherein the above composition further contains a component (D): a skin-activating component.

The present invention also provides the use of an agent for inhibiting water evaporation of a cosmetic, drug or food comprising an aqueous composition containing the following components (A) and (B) as active ingredients:
(A) a polyoxyethylene alkyl or alkenyl ether having an alkyl or alkenyl group having 20 to 24 carbon atoms and an average molar number of ethylene oxide added of 1.5 to 4; and
(B) a water-soluble polymer.

The present invention further provides a method of inhibiting water evaporation of a cosmetic, drug or food comprising an aqueous composition by incorporating a polyoxyethylene alkyl or alkenyl ether (A) having an alkyl or alkenyl group having 20 to 24 carbon atoms and an average molar number of ethylene oxide added of 1.5 to 4 and a water-soluble polymer (B) in the aqueous composition.

### [Brief Description of the Drawings]

[Figure 1] Figure 1 shows the states of pumps 30 days later in Example 8 and Comparative Example 8.
[Figure 2] Figure 2 is a graph showing changes in water content of horny layer in Test 8.
[Figure 3] Figure 3 is a graph showing changes in transepidermal water loss in Test 8.

### [Embodiments for carrying out the Invention]

The present invention relates to provision of a cosmetic, drug or food contained in a container comprising an aqueous composition, where water evaporation is inhibited to prevent the contents from solidifying or changing in properties and to show excellent long-term stability. Even when the container is a spray container or a pump container, the contents adhering to the discharge opening of the container is inhibited from drying, and thereby clogging can be prevented.

The present invention also relates to provision of a composition giving a refreshing feeling without a sticky feeling when it is applied to the skin, having high compatibility with the skin, and also having excellent occluding properties.

Accordingly, the present inventor has searched components that can inhibit water evaporation of an aqueous composition and have found that a combination of a water-soluble polymer and a polyoxyethylene alkyl or alkenyl ether can considerably inhibit water evaporation of an aqueous composition and that the effect thereof is particularly excellent in the case of using a polyoxyethylene alkyl or alkenyl ether having an alkyl or alkenyl group having 20 to 24 carbon atoms and an average molar number of ethylene oxide added of 1.5 to 4.

It was found that when an aqueous composition containing such a specific polyoxyethylene alkyl or alkenyl ether and a water-soluble polymer is applied to the skin, water evaporation is inhibited by interaction of the hydrophobic group (alkyl or alkenyl group) and the hydrophilic group (ethylene oxide group) of the polyoxyethylene alkyl or alkenyl ether to give excellent occluding properties. In addition, it was found that when a skin-activating component such as a plant extract or γ-amino-β-hydroxybutyric acid is added to the aqueous composition, the effects of these components are synergistically enhanced.

The aqueous composition as used according to the present invention has a high water-evaporation inhibiting effect, can inhibit a change in appearance, such as separation, even in long-period storage, can have excellent long-term stability, and can maintain the initial characteristics for a long time. The aqueous composition expresses a high water-evaporation inhibiting effect by containing a small amount of the polyoxyethylene alkyl or alkenyl ether and can therefore be used in any formulation and does not affect the sense of use.

The aqueous composition as used according to the present invention, when it is applied to the skin, shows a high water-evaporation inhibiting effect and an excellent occluding effect of the coating after the application thereof. The aqueous composition stably holds water on the skin for a long time and thereby enhances permeability of the skin-activating component such as a plant extract or γ-amino-β-hydroxybutyric acid into the skin to synergistically enhance the effects possessed by the skin-activating component, such as moisturizing effect and anti-inflammatory effect.

Preferred embodiments according to the present invention are, for example, as follows:
[1] An aqueous composition contained in a container, containing the following components (A), (B), and (C):
   (A) a polyoxyethylene alkyl or alkenyl ether having an alkyl or alkenyl group having 20 to 24 carbon atoms and an average molar number of ethylene oxide added of 1.5 to 4,
   (B) a water-soluble polymer, and
   (C) water;
[2] An agent for inhibiting water evaporation of an aqueous composition that contains the following components (A) and (B) as active ingredients:
   (A) a polyoxyethylene alkyl or alkenyl ether having an alkyl or alkenyl group having 20 to 24 carbon atoms and an average molar number of ethylene oxide added of 1.5 to 4; and
   (B) a water-soluble polymer;
[3] A method of inhibiting water evaporation of a cosmetic, drug or food comprising an aqueous composition by incorporating a polyoxyethylene alkyl or alkenyl ether (A) having an alkyl or alkenyl group having 20 to 24 carbon atoms and an average molar number of ethylene oxide added of 1.5 to 4 and a water-soluble polymer (B) in the aqueous composition;
[4] The cosmetic, drug or food contained in a container according to embodiment [1], the use of the agent for inhibiting water evaporation according to embodiment [2], or the method of inhibiting water evaporation according to embodiment [3], wherein component (A) is a polyoxyethylene alkyl or alkenyl ether having an alkyl or alkenyl group having 20 to 24 carbon atoms and an average molar number of ethylene oxide added of 1.5 to 3;
[5] The cosmetic, drug or food contained in a container according to embodiment [1] or [4], the use of the agent for inhibiting water evaporation according to embodiment [2] or [4], or the method of inhibiting water evaporation according to embodiment [3] or [4], wherein component (A) is a polyoxyethylene alkyl or alkenyl ether having an alkyl or alkenyl group having 20 to 24 carbon atoms and an average molar number of ethylene oxide added of 1.5 to 2.5;
[6] The cosmetic, drug or food contained in a container according to any one of embodiments [1], [4], and [5], the use of the agent for inhibiting water evaporation according to any one of embodiments [2], [4], and [5], or the method of inhibiting water evaporation according to any one of embodiments [3], [4], and [5], wherein component (A) is selected from the group consisting of polyoxyethylene(2) aralkyl ether, polyoxyethylene(3) aralkyl ether, polyoxyethylene(4) aralkyl ether, polyoxyethylene(2) behenyl ether, polyoxyethylene(3) behenyl ether, polyoxyethylene(4) behenyl ether, polyoxyethylene(2) carnaval ether, polyoxyethylene(3) carnaubyl ether, and polyoxyethylene(4) carnaubyl ether;
[7] The cosmetic, drug or food contained in a container according to any one of embodiments [1] and [4] to [6], the use of the agent for inhibiting water evaporation according to any one of embodiments [2] and [4] to [6], or the method of inhibiting water evaporation according to any one of embodiments [3] and [4] to [6], wherein component (A) is a polyoxyethylene alkyl or alkenyl ether having an alkyl or alkenyl group having 21 to 23 carbon atoms and an average molar number of ethylene oxide added of 1.5 to 4;
[8] The cosmetic, drug or food contained in a container according to any one of embodiments [1] and [4] to [7], the use of the agent for inhibiting water evaporation according to any one of embodiments [2] and [4] to [7], or the method of inhibiting water evaporation according to any one of embodiments [3] and [4] to [7], wherein component (A) is a polyoxyethylene alkyl or alkenyl ether having an alkyl or alkenyl group having 21 to 23 carbon atoms and an average molar number of ethylene oxide added of 1.5 to 3;
[9] The a cosmetic, drug or food contained in a container according to any one of embodiments [1]and [4] to [8], the use of the agent for inhibiting water evaporation according to any one of embodiments [2] and [4] to [8], or the method of inhibiting water evaporation according to any one of embodiments [3] and [4] to [8], wherein component (A) is a polyoxyethylene alkyl or alkenyl ether having an alkyl or alkenyl group having 21 to 23 carbon atoms and an average molar number of ethylene oxide added of 1.5 to 2.5;
[10] The cosmetic, drug or food contained in a container according to any one of embodiments [1] and [4] to [9], the use of the agent for inhibiting water evaporation according to any one of embodiments [2] and [4] to [9], or the method of inhibiting water evaporation according to any one of embodiments [3] and [4] to [9], wherein component (A) is polyoxyethylene(2) behenyl ether;
[11] The cosmetic, drug or food contained in a container according to any one of embodiments [1] and [4] to [10], the use of the agent for inhibiting water evaporation according to any one of embodiments [2] and [4] to [10], or the method of inhibiting water evaporation according to any one of embodiments [3] and [4] to [10], wherein the content of component (A) is 0.05 to 20% by mass based on the total amount of the composition;
[12] The cosmetic, drug or food contained in a container according to any one of embodiments [1] and [4] to [11], the use of the agent for inhibiting water evaporation according to any one of embodiments [2] and [4] to [11], or the method of inhibiting water evaporation according to any one of embodiments [3] and [4] to [11], wherein the content of component (A) is 0.1 to 20% by mass based on the total amount of the composition;
[13] The cosmetic, drug or food contained in a container according to any one of embodiments [1] and [4] to [12], the use of the agent for inhibiting water evaporation according to any one of embodiments [2] and [4] to [12], or the method of inhibiting water evaporation according to any one of embodiments [3] and [4] to [12], wherein the content of component (A) is 0.1 to 10% by mass based on the total amount of the composition;
[14] The cosmetic, drug or food contained in a container according to any one of embodiments [1]and [4] to [13], the use of the agent for inhibiting water evaporation according to any one of embodiments [2] and [4] to [13], or the method of inhibiting water evaporation according to any one of embodiments [3] and [4] to [13], wherein component (B) is one or more selected from the group consisting of carboxyvinyl polymers, alkyl acrylate/methacrylate copolymers, xanthan gum, hydroxypropyl methylcellulose, polyacrylamides, and (sodium acrylate/sodium acryloyl dimethyl taurate) copolymers;
[15] The cosmetic, drug or food contained in a container according to any one of embodiments [1] and [4] to [14], the use of the agent for inhibiting water evaporation according to any one of embodiments [2] and [4] to [14], or the method of inhibiting water evaporation according to any one of embodiments [3] and [4] to [14], wherein the content of component (B) is 0.01 to 5% by mass based on the total amount of the composition;
[16] The cosmetic, drug or food contained in a container according to any one of embodiments [1] and [4] to [15], the use of the agent for inhibiting water evaporation according to any one of embodiments [2] and [4] to [15], or the method of inhibiting water evaporation according to any one of embodiments [3] and [4] to [15], wherein the content of component (B) is 0.05 to 3% by mass based on the total amount of the composition;
[17] The cosmetic, drug or food contained in a container according to any one of embodiments [1] and [4] to [16], wherein the content of component (C) is 10% by mass or more based on the total amount of the composition;
[18] The cosmetic, drug or food contained in a container according to any one of embodiments [1] and [4] to [17], wherein the content of component (C) is 15% by mass or more based on the total amount of the composition;
[19] The cosmetic, drug or food contained in a container according to any one of embodiments [1] and [4] to [18], wherein the content of component (C) is 20% by mass or more based on the total amount of the composition;
[20] The cosmetic, drug or food contained in a container according to any one of embodiments [1] and [4] to [19], the use of the agent for inhibiting water evaporation according to any one of embodiments [2] and [4] to [16], or the method of inhibiting water evaporation according to any one of embodiments [3] and [4] to [16], wherein the aqueous composition further contains a polyol;
[21] The cosmetic, drug or food contained in a container according to embodiment [20], the use of the agent for inhibiting water evaporation according to embodiment [20], or the method of inhibiting water evaporation according to embodiment [20], wherein the polyol is selected from the group consisting of a glycol, glycerine, diglycerine, and polyglycerine.
[22] The cosmetic, drug or food comprising contained in a container according to any one of embodiments [1] and [4] to [21], the use of the agent for inhibiting water evaporation according to any one of embodiments [2], [4] to [16], [20], and [21], or the method of inhibiting water evaporation according to any one of embodiments [3], [4] to [16], [20], and [21], wherein the aqueous composition is in a formulation of an aqueous solution or an emulsion having an aqueous phase as a continuous phase;
[23] The cosmetic, drug or food contained in a container according to any one of embodiments [1] and [4] to [22], the use of the agent for inhibiting water evaporation according to any one of embodiments [2], [4] to [16], and [20] to [22], or the method of inhibiting water evaporation according to any one of embodiments [3], [4] to [16], and [20] to [22], wherein the aqueous composition has a viscosity (at 25°C) of 100 mPa·s or less;
[24] The a cosmetic, drug or food contained in a container according to any one of embodiments [1] and [4] to [23], the use of the agent for inhibiting water evaporation according to any one of embodiments [2], [4] to [16], and [20] to [23], or the method of inhibiting water evaporation according to any one of embodiments [3], [4] to [16], and [20] to [23], wherein the aqueous composition further contains a component (D): a skin-activating component;
[25] The cosmetic, drug or food contained in a container according to embodiment [24], the use of the agent for inhibiting water evaporation according to embodiment [24], or the method of inhibiting water evaporation according to embodiment [24], wherein component (D) is a plant extract;
[26] The cosmetic, drug or food contained in a container according to embodiment [25], the use of the agent for inhibiting water evaporation according to embodiment [25], or the method of inhibiting water evaporation according to embodiment [25], wherein the plant extract (D) is one or more selected from the group consisting of extracts of plants belonging to the family Brassicaceae, Poaceae, Clusiaceae, Scrophulariaceae, Zingiberaceae, Caprifoliaceae, Rosaceae, Leguminosae, Rutaceae, Magnoliaceae, and Saxifragaceae and seaweeds;
[27] The cosmetic, drug or food contained in a container according to embodiment [25] or [26], the use of the agent for inhibiting water evaporation according to embodiment [25] or [26], or the method of inhibiting water evaporation according to embodiment [25] or [26], wherein the content of the plant extract (D) is 0.0001 to 5% by mass in terms of solid content based on the total amount of the composition;
[28] The cosmetic, drug or food contained in a container according to any one of embodiments [24] to [27], the use of the agent for inhibiting water evaporation according to any one of embodiments [24] to [27], or the method of inhibiting water evaporation according to any one of embodiments [24] to [27], wherein the content of the plant extract (D) is 0.002 to 3% by mass in terms of solid content based on the total amount of the composition;
[29] The cosmetic, drug or food contained in a container according to any one of embodiments [24] to [28], the use of the agent for inhibiting water evaporation according to any one of embodiments [24] to [28], or the method of inhibiting water evaporation according to any one of embodiments [24] to [28], wherein the content of the plant extract (D) is 0.003 to 1% by mass in terms of solid content based on the total amount of the composition;
[30] The cosmetic, drug or food contained in a container according to embodiment [24], the use of the agent for inhibiting water evaporation according to embodiment [24], or the method of inhibiting water evaporation according to embodiment [24], wherein component (D) is one or more selected from the group consisting of anti-inflammatory agents, moisturizing agents, amino acids, whitening agents, singlet oxygen quenchers, antioxidants, blood circulation accelerators, sebum secretion inhibitors, antibacterial agents, and keratolytic agents;
[31] The cosmetic, drug or food contained in a container according to embodiment [30], the use of the agent for inhibiting water evaporation according to embodiment [30], or the method of inhibiting water evaporation according to embodiment [30], wherein component (D) is one or more selected from the group consisting of γ-amino-β-hydroxybutyric acid or salts thereof, γ-aminobutyric acid or salts thereof, L-carnitine or salts thereof, glycyrrhizic acid or salts thereof, glycyrrhetinic acid or salts thereof, isopropyl aminocaproic acid or salts thereof, allantoin, lysozyme chloride, guaiazulene, methyl salicylate, γ-orizanol, N-methyl-L-serine, chondroitin sulfate or salts thereof, collagen (average molecular weight: 50000 to 6000000), water-soluble collagen, hydrolyzed collagen (average molecular weight: 100 to 9000), royal jelly extracts, lactic acid and salts thereof, elastin, chitin, chitosan, urea, pyrrolidonecarboxylic acid or salts thereof, amino acids or salts thereof, L-ascorbic acid or derivatives thereof, rhododendrol or derivatives thereof, arbutin, tranexamic acid or derivatives thereof, 4-methoxysalicylic acid or salts thereof, carotenoids such as α-carotene, β-carotene, γ-carotene, lycopene, cryptoxanthin, lutein, zeaxanthin, isozeaxanthin, rhodoxanthin, capsanthin, and crocetin, 1,4-diazacyclooctane, 2,5-dimethylfuran, 2-methylfuran, 2,5-diphenylfuran, 1,3-diphenylisobenzofuran, α-tocopherol, β-tocopherol, γ-tocopherol, d-tocopherol, histidine, tryptophan, methionine, alanine, or alkyl esters thereof, dibutylhydroxytoluene, butylhydroxyanisole, ascorbic acid, tannic acid, epicatechin, epigallocatechin, epicatechin gallate, epigallocatechin gallate, flavonoid, α-tocopherol, β-tocopherol, γ-tocopherol, d-tocopherol, nicotinamide, methyl nicotinate, oxendolone, 17-α-methyl-β-nortestosterone, chlormadinone acetate, cyproterone acetate, spironolactone, hydroxyflutamide, estradiol, ethinylestradiol, zinc sulfocarbolate, zinc oxide, aluminum hydroxychloride, allantoin dihydroxyaluminum, vitamin B6, 13-cis-retinoic acid, vitamin E, glycyrrhetinic acid, salicylic acid, nicotinic acid, calcium pantothenate, dipotassium azelate, 10-hydroxyundecanoic acid, 12-hydroxystearic acid, sulfur, triclosan, trichlorocarbanilide, chlorhexine hydrochloride, benzalkonium chloride, benzethonium chloride, cetylpyridinium chloride, benzalkonium alkylphosphate, isopropyl methylphenol, benzoic acid, photosensitizer No. 201, resorcin, salicylic acid, N-acetylcysteine, lactic acid, citric acid, succinic acid, malic acid, alkylamine oxide, and benzoyl peroxide;
[32] The cosmetic, drug or food contained in a container according to embodiment [30] or [31], the use of the agent for inhibiting water evaporation according to embodiment [30] or [31], or the method of inhibiting water evaporation according to embodiment [30] or [31], wherein component (D) is one or more selected from the group consisting of γ-amino-β-hydroxybutyric acid or salts thereof, glycyrrhizic acid or salts thereof, γ-aminobutyric acid or salts thereof, L-carnitine or salts thereof, rhododendrol, N-methyl-L-serine, niacinamide, ascorbic acid-2-glucoside, and isopropyl methylphenol;
[33] The cosmetic, drug or food contained in a container according to any one of embodiments [1] and [4] to [32], the use of the agent for inhibiting water evaporation according to any one of embodiments [2], [4] to [16], and [20] to [32], or the method of inhibiting water evaporation according to any one of embodiments [3], [4] to [16], and [20] to [32], wherein the content of component (A) is 0.05 to 20% by mass based on the total amount of the composition, and the content of component (B) is 0.01 to 5% by mass based on the total amount of the composition;
[34] The cosmetic, drug or food contained in a container according to any one of embodiments [1] and [4] to [33], the use of the agent for inhibiting water evaporation according to any one of embodiments [2], [4] to [16], and [20] to [33], or the method of inhibiting water evaporation according to any one of embodiments [3], [4] to [16], and [20] to [33], wherein the content of component (A) is 0.1 to 20% by mass based on the total amount of the composition, and the content of component (B) is 0.05 to 3% by mass based on the total amount of the composition;
[35] The cosmetic, drug or food contained in a container according to any one of embodiments [1] and [4] to [34], the use of the agent for inhibiting water evaporation according to any one of embodiments [2], [4] to [16], and [20] to [34], or the method of inhibiting water evaporation according to any one of embodiments [3], [4] to [16], and [20] to [34], wherein the content of component (A) is 0.1 to 10% by mass based on the total amount of the composition, and the content of component (B) is 0.05 to 3% by mass based on the total amount of the composition;
[36] The cosmetic, drug or food contained in a container according to any one of embodiments [1] and [4] to [35], the use of the agent for inhibiting water evaporation according to any one of embodiments [2], [4] to [16], and [20] to [35], or the method of inhibiting water evaporation according to any one of embodiments [3], [4] to [16], and [20] to [35], wherein the aqueous composition is an aqueous composition for use as a cosmetic, drug, quasi-drug, or food; and
[37] The cosmetic, drug or food contained in a container according to any one of embodiments [1] and [4] to [36], the use of the agent for inhibiting water evaporation according to any one of embodiments [2], [4] to [16], and [20] to [36], or the method of inhibiting water evaporation according to any one of embodiments [3], [4] to [16], and [20] to [36], wherein the aqueous composition is a composition for external use on skin.

The constitution of the present invention will now be described.

The aqueous composition in the present invention is a composition having an aqueous phase as a continuous phase, and encompasses a water-soluble composition containing a water-soluble component, an oil-in-water emulsion composition containing water in the continuous phase, and a W/O/W emulsion composition.

The aqueous phase in the present invention is a phase containing water.

In the polyoxyethylene alkyl or alkenyl ether (A) used in the present invention, the alkyl or alkenyl group has 20 to 24 carbon atoms, and the average molar number of ethylene oxide added is 1.5 to 4.

The alkyl or alkenyl group of the polyoxyethylene alkyl ether may be a linear chain or a branched chain without limiting the structure thereof, but is preferably a linear or branched alkyl group and more preferably a linear alkyl group. The alkyl or alkenyl group has 20 to 24 carbon atoms, preferably 21 to 23 carbon atoms, and most preferably 22 carbon atoms, i.e., a behenyl group. An alkyl or alkenyl group having less than 20 carbon atoms has a low water-evaporation inhibiting effect and is therefore not preferred. An alkyl or alkenyl group having higher than 24 carbon atoms is difficult to be dissolved in an aqueous phase and is therefore not preferred from the viewpoint of formulation.

The alkyl or alkenyl ether has an average molar number of ethylene oxide added in the range of 1.5 to 4, preferably 1.5 to 3, and most preferably 1.5 to 2.5. An average molar number of ethylene oxide added of less than 1.5 causes high crystallizability and difficulty in dissolving in an aqueous phase and is therefore not preferred. An average molar number of ethylene oxide added of higher than 4 notably decreases the water-evaporation inhibiting effect and is therefore not preferred. Polyoxyethylene alkyl or alkenyl ethers that can be generally available are each a mixture having a significantly broad distribution of the molar number of ethylene oxide added with a desired degree of polymerization at the center, but it is important in the present invention to have the average molar number of ethylene oxide added in the above-mentioned range.

Examples of the polyoxyethylene alkyl or alkenyl ether (hereinafter may be simply referred to as polyoxyethylene alkyl ether or POE alkyl ether) used in the present invention include polyoxyethylene(2) arachyl ether, polyoxyethylene(3) arachyl ether, polyoxyethylene(4) arachyl ether, polyoxyethylene(2) behenyl ether, polyoxyethylene(3) behenyl ether, polyoxyethylene(4) behenyl ether, polyoxyethylene(2) carnaubyl ether, polyoxyethylene(3) carnaubyl ether, and polyoxyethylene(4) carnaubyl ether; preferably polyoxyethylene(2) behenyl ether, polyoxyethylene(3) behenyl ether, and polyoxyethylene(4) behenyl ether; and more preferably polyoxyethylene(2) behenyl ether. In addition, a polyoxyethylene alkyl ether other than those mentioned above may be concomitantly used as long as the polyoxyethylene alkyl ether has an average molar number of ethylene oxide added in the above-mentioned range.

The content of the polyoxyethylene alkyl ether in the aqueous composition as used according to the present invention is preferably 0.05 to 20% by mass, more preferably 0.1 to 20% by mass, and even more preferably 0.1 to 10% by mass, based on the total amount of the composition. A content within the range can provide high water-evaporation inhibiting effect and occluding effect and can be easily blended into an aqueous phase and is therefore preferred.

The aqueous composition as used according to the present invention contains a water-soluble polymer (B). The water-soluble polymer allows the polyoxyethylene alkyl ether (A) to stably disperse in an aqueous phase and contributes to the water-evaporation inhibiting effect.

Examples of the water-soluble polymer used in the present invention include water-soluble cationic, anionic, nonionic, amphoteric, and bipolar polymers.

Specific examples of the cationic polymers include poly(dimethyl diallyl ammonium halide) cationic polymers, cationic copolymers of dimethyl diallyl ammonium halide and acrylamide, quaternary nitrogen-containing cellulose ether, cationized products of vinylpyrrolidone/dimethylaminoethyl methacrylate copolymers, and condensation products of polyethylene glycol, epichlorohydrin, propylene amine, and tallowyl or cocoyl amine obtained from beef tallow fatty acids. Commercially available examples of the poly(dimethyl diallyl ammonium halide) cationic polymers include Merquat 100, a trade name, available from Merck & Co., Inc., U.S.A. Commercially available examples of the cationic copolymers of dimethyl diallyl ammonium halide and acrylamide include Merquat 550 (Merquat & Co., Inc., U.S.A.). Commercially available examples of the condensation products of polyethylene glycol, epichlorohydrin, propylene amine, and tallowyl or cocoyl amine include Polyquat H, a trade name, available from Henkel International Co., Germany. Commercially available examples of the quaternary nitrogen-containing cellulose ether include Polymer JR-400, Polymer JR-125, and Polymer JR-30M, trade names, available from Union Carbide Corp., U.S.A. Commercially available examples of the cationized products of vinylpyrrolidone/dimethylaminoethyl methacrylatecopolymer include Gafquat 755 and Gafquat 734, trade names, available from GAF Corp., U.S.A.

Specific examples of the anionic polymers include carboxyvinyl polymers, carboxymethyl cellulose, carageenan, xanthan gum, polystyrene sulfonate, agar, Gatti gum, karaya gum, pectin, alginate salts, poly(acrylic acid), and acrylic or methacrylic acid derivatives such as alkali metal salts and ammonium salts of acrylic acid and methacrylic acid.

Specific examples of the nonionic polymers include cellulose ethers (e.g., hydroxybutyl methylcellulose, hydroxypropyl cellulose, hydroxypropyl methylcellulose, ethylhydroxyethyl cellulose, and hydroxyethyl cellulose), propylene glycol alginate, polyacrylamide, (sodium acrylate/sodium acryloyl dimethyl taurate) copolymers, poly(ethylene oxide), polyvinyl alcohol, polyvinylpyrrolidone, hydroxypropyl guar gum, locust bean gum, amylose, hydroxyethyl amylose, hyaluronic acid or alkali metal salts thereof, starch, starch derivatives, and mixtures thereof.

Specific examples of the amphoteric polymers and the bipolar polymers include octyl acrylamide/acrylate/butylaminoethyl methacrylate copolymers, polyquaternium-47, and polyquaternium-43.

These water-soluble polymers may be used alone or in combination thereof. Preferred from the viewpoint of application to various formulations are carboxyvinyl polymers, alkyl acrylate/methacrylate copolymers, xanthan gum, hydroxypropyl methylcellulose, polyacrylamide, (sodium acrylate/sodium acryloyl dimethyl taurate) copolymers, and hyaluronic acid and alkali metal salts thereof.

The content of the water-soluble polymer used in the present invention is preferably 0.01 to 5% by mass, more preferably 0.05 to 3% by mass, based on the total amount of the composition. A content within the range can maintain stability of a pharmaceutical preparation and provide excellent water-evaporation inhibiting effect and occluding effect and is therefore preferred.

The aqueous composition as used according to the present invention preferably further contains a polyol in order to enhance the solubility of the polyoxyethylene alkyl ether in an aqueous phase. Preferred examples of the polyol include glycols such as ethylene glycol, diethylene glycol, triethylene glycol, polyethylene glycol (average molecular weight: less than 1000), propylene glycol, dipropylene glycol, polypropylene glycol (average molecular weight: less than 1000), isoprene glycol, and 1,3-butylene glycol; and glycerine, diglycerine, and polyglycerine.

The content of the water (C) in the aqueous composition as used according to the present invention is not limited, as long as the content is within the range that can form an aqueous solution or an emulsion having a continuous phase of an aqueous phase such as an O/W system or a W/O/W system, and is preferably 10% by mass or more, more preferably 15% by mass or more, and even more preferably 20% by mass or more.

Even if the aqueous composition as used according to the present invention is a liquid having a low viscosity (at 25°C) of 100 mPa·s or less, a high water-evaporation inhibiting effect can be provided as long as the stability of a pharmaceutical preparation is maintained.

The aqueous composition as used according to the present invention can further contain a skin-activating component (D) and can enhance the effect of the skin-activating component by a synergistic effect of the skin-occluding effect and the effect of the skin-activating component.

The skin-activating component as component (D) used in the present invention shows some activity on the skin, such as a moisturizing effect, a skin-softening effect, a whitening effect, an anti-inflammatory effect, an antioxidant effect, a blood circulation-accelerating effect, an anti-aging effect (anti-wrinkle effect), and a sebum secretion-inhibiting effect. Such an activating component is not particularly limited, as long as the activating component may be any activating component that is usually used in cosmetics, drugs, etc. Examples of the activating component include plant extracts, anti-inflammatory agents, moisturizing agents, amino acids, whitening agents, singlet oxygen quenchers, antioxidants, blood circulation accelerators, sebum secretion inhibitors, antibacterial agents, and keratolytic agents.

As described above, use of a combination of a water-soluble polymer and a polyoxyethylene alkyl or alkenyl ether having an alkyl or alkenyl group having 20 to 24 carbon atoms and an average molar number of ethylene oxide added of 1.5 to 4 shows interaction of the hydrophobic group (alkyl or alkenyl group) and the hydrophilic group (ethylene oxide group) of the polyoxyethylene alkyl or alkenyl ether to inhibit water evaporation, express excellent occluding properties, and enhance the permeation of the skin-activating component (D). As a result, the effects of the skin-activating component, such as a moisturizing effect, an effect of preventing or improving rough skin, an effect of preventing or improving deterioration in skin tension and elasticity and dullness of complexion, an effect of preventing or improving outstanding pores and acne caused by excess sebum secretion, bacteria, or keratonosis, an effect of preventing or improving wrinkle formation, an effect of preventing or improving spots and freckles, an effect of preventing or improving outstanding pores and acne caused by excess sebum secretion, and an effect of preventing or improving wrinkle formation, are synergistically enhanced, and also a composition for external use on skin having excellent sense of use can be obtained.

Among these skin-activating components, the present invention preferably uses a hydrophilic skin-activating component because of its high water-evaporation inhibiting effect, which is caused by that the hydrophilic skin-activating component is retained on the skin for a long time and thereby that the permeability of the skin-activating component is enhanced.

Specific examples of the skin-activating component (D) will now be described. Components having multiple functions may be redundantly described. These are mere exemplification and the component (D) is not limited thereto.

The plant extract as the skin-activating component (D) used in the present invention is not particularly limited, as long as it may be any plant extract that shows a medicinal effect on the skin. As a plant extract having excellent properties of retaining moisture and tension of the skin, it is preferable to use one or more extracts selected from extracts of plants belonging to the family Brassicaceae, Poaceae, Clusiaceae, Scrophulariaceae, Zingiberaceae, Caprifoliaceae, Rosaceae, Leguminosae, Rutaceae, Magnoliaceae, and Saxifragaceae and seaweeds.

In the present invention, the part of a plant used is not particularly limited, and aerial parts including flowers, stems, leaves, seeds, fruits, and pericarps and underground parts including roots and underground stems may be used alone or in a mixture thereof. Extraction thereof may be performed by using the plant itself or using a processed product by, for example, drying, chopping, or pulverizing.

The solvent used for plant extraction may be a polar solvent or a non-polar solvent. Examples of the polar solvent include water and organic solvents such as lower alcohol, higher alcohol, hydrocarbons, and derivatives thereof. Examples of the non-polar solvent include petroleum ether, aliphatic hydrocarbons having 4 to 8 carbon atoms, halides of aliphatic hydrocarbons having 1 or 2 carbon atoms, and aromatic hydrocarbons having 6 to 7 carbon atoms. The method for extraction is not particularly limited. Among these plant extracts, preferred are those extracted with a polar solvent, in particular, with water or a mixture of water and lower alcohol such as methanol, ethanol, or isopropanol. In such a case, the ratio of the lower alcohol to water, i.e., lower alcohol/water, is preferably from 0/100 to 70/30 (V/V: volume ratio), more preferably from 0/100 to 40/60.

The thus-prepared plant extract may be used directly, or used as a diluted solution or as a concentrated extract. Furthermore, the extract may be prepared into a dried powder form by, for example, lyophilization, or prepared into a paste form. Furthermore, the extract may be used as a fermentation product. In the case of a dried powder, the powder is preferably used after being dissolved in water or lower alcohol, such as methanol, ethanol, or isopropanol, containing water or used by solubilization in the composition containing water.

Plants belonging to the family Brassicaceae are not particularly limited, and preferred examples thereof include those belonging to the genus Brassica, such as *taasai* (B. campestris var. narinosa), Indian mustard (B. juncea), Brown mustard (B. juncea), *takana* (B. juncea var. integlifolia), Swede Gowrie (B. napobrassica), Rapeseed (B. napus), Potherb mustard (B. nipposinica), Black mustard (B. nigra), Kale (B. oleracea var. acephala), Ornamental cabbage (B. oleracea var. acephala), Chinese kale (B. oleracea var. allbograbra), Cauliflower (B. oleracea var. botrytis), Romanesco broccoli (B. oleracea var. botrytis), Cabbage (B. oleracea var. capitata), Brussels sprouts (B. oleracea var. gemmifera), Kohlrabi (B. oleracea var. gongylodes), Broccoli (B. oleracea var. italic), Green pak choi (B. rapa var. chinensis), Pak choi (B. rapa var. chinensis), *nozawana* (B. rapa var. hakabura), *mibuna* (B. rapa var. lancinifolia), natane (B. rapa var. nippo-oleifera), Chinese cabbage (B. rapa var. pekinensis), Komatsuna (B. rapa var. peruviridis), and Turnip (B. rapa var. rapa); those belonging to the genus Camelina, such as Camelina alyssum (C. sativa); those belonging to the genus Matthiola, such as Stock (M. incana); those belonging to the genus Ionopsidium, such as Violet cress (I. acaule); those belonging to the genus Rorippa, such as *mitibatagarashi* (R. dubia), Variableleaf yellowcress (R. indica), and Bog yellowcress (R. islandica); those belonging to the genus Draba, such as *hariinunazuna* (D. aizoidas) and *inunazuna* (D. nemorosa); those belonging to the genus Aurinia, such as Golden alyssum (A. saxatilis); those belonging to the genus Diplotaxis, such as Wild rucolla (D. muralis); those belonging to the genus Orychophragmus, such as *ooaraseitou* (O. violaceus); those belonging to the genus Nasturtium, such as Watercress (N. officinale); those belonging to the genus Coronopus, such as White snakeroot (C. didymus) and *hamagarashi* (C. integrifolis); those belonging to the genus Eruca, such as Garden rocket (E. vesicaria); those belonging to the genus Sisymbrium, such as *hosoegarashi* (S. irio), *hokobagarashi* (S. loeselii), *kakinegarashi* (S. officinale), and *inukakinegarashi* (S. orientale); those belonging to the genus Thlaspi, such as Field Penny-cress (T. arvense); those belonging to the genus Lunaria, such as Satinpod (L. annua); those belonging to the genus Arabidopsis, such as Thale cress (A. thaliana); those belonging to the genus Armoracia, such as Horseradish (A. rusticana); those belonging to the genus Hirschfedia, such as *daikonmodoki* (H. incana); those belonging to the genus Raphanus, such as Japanese radish (R. sativus), Radish (R. s. var. radicula), and *hamadaikon* (R. s. var. raphanistroides); those belonging to the genus Cardamine, such as Bitter cress (C. flexuosa), Narrow-leaved bittercress (C. impatiens), and *himetanetsukebana* (C. parviflora); those belonging to the genus Capsella, such as Shepherd's purse (C. bursa-pastoris); those belonging to the genum Lobularia, such as Sweet alyssum (L. maritima); those belonging to the genus Hesperis, such as Dame's rocket (H. matronalis); those belonging to the genus Iberis, such as *iberis amara* (I. amara), *tokiwanazuna* (I. sempervirens), and candytuft (I. umbellata); those belonging to the genus Lepidium, such as *himegunbaizanuna* (L. apetalum), Maca (L. meyenii), *koshiminonazuna* (L. perfoliatum), Peppergrass (L. sativum), and Virginia pepperweed (L. virginicum); those belonging to the genus Malcolmia, such as Virginia stock (M. maritima); those belonging to the genus Rapistrum, such as Turnip weed (R. rugosum); those belonging to the genus Alyssum, such as *miyamanazuna* (A. alpestre), Yellow alyssum (A. alyssoides), and *yamanazuna* (A. montanum); and those belonging to the genus Wasabia, such as wasabi (W. japonica).

Among these plants belonging to the family Brassicaceae, preferred are plants belonging to the genus Brassica, Nasturtium, Lepidium, Armoracia, or Wasabia.

Plants belonging to the family Poaceae are not particularly limited, and preferred examples thereof include those belonging to the genus Agropyron, such as Couch grass (A. repens), *aokamozigusa* (A. ciliare var. minus), and *kamozigusa* (A. tsukushiense var. transiens); those belonging to the genus Horudeum, such as Barley (H. vulgare) and Wall barley (H. murinum); those belonging to the genus Triticum, such as wheat (T. aestivum) and Durum wheat (T. durum); those belonging to the genus Secale, such as Rye (S. cereale); those belonging to the genus Avena, such as Oat (A. sativa) and *hadakaenbaku* (A. nuda); those belonging to the genus Oryza, such as Rice (O. sativa); those belonging to the genus Coix, such as Large-fruited adlay (C. lacryma-jobi var. ma-yuen); those belonging to the genus Miscanthus, such as Amur silver-grass (M. sacchariflorus) and Silver grass (M. sinensis); those belonging to the genus Panicum, such as *nukakibi* (P. bisulcatum) and *houkinukakibi* (P. scoparium); those belonging to the genus Phragmites, such as Reed canary grass (P. arundinacea) and Common reed (P. communis); those belonging to the genus Phyllostachys, such as Moso bamboo (P. pubescens); those belonging to the genus Saccharum, such as Sugarcane (S. officinarum); those belonging to the genus Sasa, such as *chimakizasa* (S. palmata); those belonging to the genus Setaria, such as Green bristlegrass (S. virides) and *hamaenokoro (S.* viridis); those belonging to the genus Zea, such as corn (Z. meys); and those belonging to the genus Zizania, such as Manchurian wild rice (Z. latifolia).

Among these plants belonging to the family Poaceae, preferred are plants belonging to the genus Triticum, Horudeum, Secale, Oryza, or Zea.

Plants belonging to the family Clusiaceae are not particularly limited, and preferred examples thereof include those belonging to the genus Hypericum, such as *otogirisou* (H. erectum), St. John's wort (H. perforatum), *biyouyanagi* (H. monogynum), and *kinshibai* (H. patulum); and those belonging to the genus Garcinia, such as mangosteen (G. mangostana), *guruguru* (G. atroviridis Griff. ex T. Anders.), *bancamangisu* (G. bancana Miq.), *kobanomangisu* (G. brevirostris Scheff.), *goraka* (G. cambogia Desr.), *cerebesumangostin* (G. celebicaLinn.), *cowaganboji* (G. cowa Roxb.), *mumungin* (G. dioicaBlume.), *oobanomangosutin* (G. dulcis. Kurz.), *akamikanzisu* (G. forbesii King.), *kanzisu* (G. globulosa Rind1.), *kiminomangisu* (G. hgriffithii T. Anders.), gamboges (G. hauburyi Hook. f.), *hamamangosutin* (G. hombroniana Pierre), *merugikanzisu* (G. merguensis Wight), *indogamubozi* (G. morella Desr.), *tsunomimangisu* (G. nigrolineata Planch. ex T. Anders.), *kobanokanzisu* (G. parvifolia Miq.), *botanmangosutin* (G. prainiana King), Common garcinia (G. subelliptica Merrill), and *kyanimomo* (G. xanthochymus Hookf.).

Among these plants belonging to the family Clusiaceae, preferred are plants belonging to the genus Hypericum or Garcinia.

Plants belonging to the family Scrophulariaceae are not particularly limited, and preferred examples thereof include those belonging to the genus Angelonia, such as *angelonia* (A. salicariifolia); those belonging to the genus Torenia, such as *tsuruurikusa* (T. concolor var. formosana) and wishbone flower (T. fournieri); those belonging to the genus Rehmannia, such as *akayajiou* (R. glutinosa Liboschitz Var. purpurea Makino), *kaikeijiou* (R. glutinosa Liboschitz), and *tenmokujiou* (R. piasezkii); and those belonging to the genus Verbascum, such as *birodomouzuika* (V. thapsus).

Among these plants belonging to the family Scrophulariaceae, preferred are those belonging to the genus Torenia or Rehmannia.

Plants belonging to the family Zingiberaceae are not particularly limited, and preferred examples thereof include those belonging to the genus Zingiber, such as ginger (Z. officinale (Willd.) Roscoe) and mioga ginger (Z. mioga (Thumb.) Roscoe); those belonging to the genus Alpinia, such as *hanamyoga* (A. Japonia (Thumb.) Miq), *aonokumatakeran* (A. intermedia Gagnep.), *iriomotekumatakeran* (A. flabellate Ridl.), *kumatakeran* (A. formosana K. Schum.), *gettou* (A. speciosa (Wendl.) K. Schum.), *shimakumatakeran* (A. boninensis Makino), *ioukumatakeran* (A. nakaiana Tuyama), and *yakuchi* (A. oxyphylla Miq.); those belonging to the genus Curcuma, such as turmeric (C. longa), zedoary (C. zedonaria Rose.), *murasakigajyutsu* (C. aeruginosa Roxb.), and *curucuma alisumatifolia* (C. alismatifolia Gagnep.); and those belonging to the genus Elettaria, such as cardamom (E. cardamomum Maton).

Among these plants belonging to the family Zingiberaceae, preferred are those belonging to the genus Zingiber, Alpinia, or Curcuma.

Plants belonging to the family Caprifoliaceae are not particularly limited, and preferred examples thereof include those belonging to the genus Abelia, such as *kotsukubaneutsugi* (A. serrate) and *tsukubaneutsugi* (A. spathulata); those belonging to the genus Lonicera, such as *nemurobushidama* (L. chrysantha var. crassipes), *uguisukagura* (L. gracilipes var. glabra), *miyamauguisukagura* (L. gracilipes var. glandulosa), Japanese honeysuckle (L. japonica), Morrow's honeysuckle (L. morrowii), and Trumpet honeysuckle (L. sempervirens); those belonging to the genus Sambucus, such as *sokuzu* (S. chinensis), European elder (S. nigra), European red elder (S. racemosa), and Japanese red elder (S. racemosa ssp. Sieboldiana); those belonging to the genus Viburnum, such as *sangojyu* (V. awabuki), Linden viburnum (V. dilatum), *kobanogamazumi* (V. erosum var. punctatum), *ookamenoki* (V. furcatum), *kanboku* (V. opulus var. calvescens), *yabudemari* (V. pilicatum var. tomentosum), *yamashigure* (V. urceolatum), and *miyamagamazumi* (V. wrghtii); those belonging to the genus Weigela, such as Crimson weigela (W. floribunda) and *taniutsugi* (W. hortensis); and those belonging to the genus Zabelia, such as *iwatsukubaneutsugi* (Z. integrifolia).

Among these plants belonging to the family Caprifoliaceae, preferred are those belonging to the genus Lonicera or Sambucus.

Plants belonging to the family Rosaceae are not particularly limited, and preferred examples thereof include those belonging to the genus Agrimonia, such as *himekinmizuhiki* (A. nipponica) and Agrimonia pilosa (A. pilosa var. japonica); those belonging to the genus Pyracantha, such as *tachibanamodoki* (P. angustifolia), Scarlet firethorn (P. coccinea), Napalese firethorn (P. crenulata), *taitousanzashi* (P. koidzumii), and Chinese firethorn (P. fortuneana); those belonging to the genus Spiraea, such as Willowleaf meadowsweet (S. salicifolia), Shiny leaf meadowsweet (S. betulifolia Pall. var. betulifolia), *ezonoshirobanashimotsuke* (S. miyabei Koidz.), Japanese meadowsweet (S. japonica), Thunberg's meadowsweet (S. thunbergii Siebold ex Blume), Bridal wreath spirea (S. prunifolia Siebold et Zucc.), *kenashiaizushimotsuke* (S. chamaedryfolia L. var. chamaedryfolia), *ezoshimotsuke* (S. media F. W. Schmidt var. sericea (Turcz.) Regel ex Maxim.), Snowmound spirea (S. nipponica Maxim. var. nipponica), *ibukishimotsuke* (S. dasyantha Bunge), Reeves' meadowsweet (S. cantoniensis Lour.), and *iwagasa* (S. blumei G. Don); those belonging to the genus Potentilla, such as English cinquefoil (P. anglica), *kawarasaiko* (P. chinensis), *tsuchiguri* (P. discolor), tormentil (P. erecta), Shrubby cinquefoil (P. fruticosa var. rigida), *miyamakinbai* (P. matsumurae), *meakakinbai* (P. miyabie), and Five-leaf cinquefoil (P. sundaica var. robusta); those belonging to the genus Fragaria, such as *nougouichigo* (F. iinumae), *shirobananohebiichigo* (F. nipponica), *benibanahebiichigo* (F. nipponica f. rosea), *yakushimashirobanahebiichigo* (F. nipponica var. yakusimensis), wild strawberry (F. vesca), *ezokusahebiichigo* (F. yezoensis), strawberry (F. ananassa), beach strawberry (F. chiloensis), and virginia strawberry (F. virginiana); those belonging to the genus Geum, such as *miyamadaikonsou* (G. calthaefolium var. nipponicum), Aleutian avens (G. pentapetalum), Japanese avens (G. japonicum), and Alpine avens (G. montanum); those belonging to the genus Duchesnea, such as *hebiichigo* (D. chrysantha) and Indian strawberry (D. indica); those belonging to the genus Rubus, such as winter berry (R. buergeri), cloudberry (R. chamaemorus), Korean raspberry (R. crataegifolius), *biroudoichigo* (R. corchorifolius), Shrubby blackberry (R. fruticosus), blackberry (R. spp.), *kusaichigo* (R. hirsutus), *nagabamomijiichigo* (R. palmatus var. palmatus), *nawashiroichigo* (R. parvifoius), *kobanofuyuichigo* (R. pectinellus), *hasunohaichigo* (Rhubus peltatus), *kojikiichigo* (R. sumatranus), and *kajiichigo* (R. trifidus); those belonging to the genus Sanguisorba, such as *karaitosou* (S. hakusanensis), Great Burnet (S. officinalis), *naganobonoshirowaremokou* (S. tenuifolia var. alba), and *tichimawaremokou* (S. tenuifolia var. grandiflora); those belonging to the genus Rosa, such as cabbage rose (R. centifolia (Rosaceae)), hybrid rose (R. Hybrida (Rosaceae)), Rose Fruit (R. multiflora Thunberg (Rosaceae)), Cherokee rose (Rosa laevigata), Banksia rose (Rosa banksiae), multiflora rose (Rosa multiflora), Japanese rose (Rosa rugosa), *yamaibara* (Rosa sambucina), apple rose (Rosa pomifera), musk rose (Rosa moschata), and dog rose (Rosa canina); those belonging to the genus Prunus, such as peach (P. persica batsch), apricot (P. armeniaca 1.), Japanese apricot (P. mume), hill cherry (P. jamasakura), and *edohigan* (P. pendula f. ascendens); those belonging to the genus Crataegus, such as Chinese hawthorn (C. cuneata), *oosanzashi* (C. pinnatifida), and English hawthorn (C. oxyacantha); those belonging to the genus Eriobotrya, such as loquat (E. japonica); those belonging to the genus Chaenomeles, such as quince (C. sinensis); those belonging to the genus Malus, such as apple (Malus pumila (Rosaceae)) and crabapple (M. halliana); those belonging to the genus Kerria, such as Japanese kerria (K. japonica); and those belonging to the genus Sorbus, such as mountain ash (S. alnifolia), European mountain ash (S. aucuparia), Japanese rowan (S. commixta), Japanese whitebeam (S. japonica), Japanese mountain ash (S. matsumurana), and Siberian mountain ash (S. sambucifolia).

Among these plants belonging to the family Rosaceae, preferred are those belonging to the genus Agrimonia, Sanguisorba, Crataegus, or Prunus.

Examples of plants belonging to the family Leguminosae include those belonging to the genus Glycine, such as soybean (G. max subsp. max) and wild soybean (G. max subsp. soja); those belonging to the genus Lathyrus, such as sea pea (L. japonicus), sweat pea (L. odoratus), and perrenial pea (L. latifolius); those belonging to the genus Albizia, such as silk tree (A. julibrissin); those belonging to the genus Sophora, such as shrubby sophora (S. flavewscens) and necklacepod (S. tomentosa); those belonging to the genus Strongylodon, such as jade vine (S. macrobotrys); those belonging to the genus Styphnolobium, such as Japanese pagoda tree (S. japonicum) and weeping pagoda tree (S. japonicum var. pendulum); those belonging to the genus Trifolium, such as Italian clover (T. incarnatum), red clover (T. pratense), white clover (T. repens), and Burchell's clover (T. burchellianum); those belonging to the genus Vicia, such as common vetch (Vicia angustifolia), faba bean (V. faba), hairy vetch (V. hirsute), smooth tare (V. tetrasperma), and two-leaf vetch (V. unijuga); those belonging to the genus Vigna, such as mung bean (V. radiata) and azuki bean (V. angularis); those belonging to the genus Wisteria, such as *yamafuji* (W. brachybotrys) and Japanese wisteria (W. floribunda); those belonging to the genus Arachis, such as peanut (A. hypogaea); those belonging to the genus Cicer, such as click pea (C. arietinum); those belonging to the genus Aspalathus, such as rooibos (A. linearis); those belonging to the genus Glycyrrhiza, such as licorice (G. glabra) and Chinese licorice (G. uralensis); those belonging to the genus Astragalus, such as milk vetch (A. membranaceus) and *renge* (A. sinicus); those belonging to the genus Lens, such as lentil (L. culinaris); those belonging to the genus Pisum, such as pea (P. sativum); those belonging to the genus Phaseolus, such as runner bean (P. coccineus), Lima bean (P. lunatus), and common bean (P. vulgaris); and those belonging to the genus Pueraria, such as kudzu (P. lobata) and *pueraria* (P. mirifica).

Among these plants belonging to the family Leguminosae, preferred are those belonging to the genus Glycine, Lathyrus, Vicia, Glycyrrhiza, Vigna, Pisum, or Phaseolus.

Examples of plants belonging to the family Rutaceae include those belonging to the genus Citrus, such as Satsuma orange (C. unshiu Marcov), *oobenimikan* (C. tangerina Tanaka), *kobenimikan (C.* erythrosa Tanaka), orange (C. aurantium L subsp. nobilis Makino), daidai (C. aurantium L. var. daidai Makino), citron (C. medica L), Natsudaidai (C. Natsudaidai Hayata), pumelo (C. grandis Osbeck), Yuzu (C. Junos Tanaka), ponkan (C. reticulata Blanco), lemon (C. limon Burmann), *karatachi* (C. trifoliata L), and grapefruit (C. paradisi Macf.); those belonging to the genus Fortunella, such as marumi kumquat (F. japonica swingle); those belonging to the genus Xanthoxyum, such as Japanese pepper (X. piperitum DC.); those belonging to the genus Euodia, such as *goshuyu* (Euodia rutaecarpa Hook. fil et Thoms.); those belonging to the genus Fragara, such as *inuzansyou* (F. mante churica Honda); those belonging to the genus Orixa, such as *kokusagi* (O. japonica Thunb); those belonging to the genus Skimmia, such as *miyamashikimi* (S. japonica Thunb); and those belonging to the genus Poncirus, such as trifoliate orange (P. trifoliata).

Among these plants belonging to the family Rutaceae, preferred are those belonging to the genus Citrus or Fortunella.

Examples of plants belonging to the family Magnoliaceae include those belonging to the genus Liriodendron, such as tulip tree (L. tulipifera); those belonging to the genus Magnolia, such as Yulan magnolia (M. denudata), southern magnolia (M. grandiflora), lily magnolia (M. liliflora), kobushi magnolia (M. kobus), Japanese big-leaf magnolia (M. obovata), Anise magnolia (M. salicifolia), and Star magnolia (M. stellata); and those belonging to the genus Michelia, such as *ogatamanoki* (M. compressa) and banana shrub (M. figo).

Among these plants belonging to the family Magnoliaceae, preferred are those belonging to the genus Liriodendron or Magnolia.

Examples of plant belonging to the family Saxifragaceae include those belonging to the genus Astilbe, such as *chidakesashi* (A. microphylla), *akashoma* (A. thunbergii var. thunbergii), and *toriashishoma* (A. thunbergii var. congesta); those belonging to the genus Bergenia, such as elephant's ear (B. stracheyi), *ganhakusai* (B. emeiensis), heartleaf bergenia (B. cordifolia), *koyoganhakusai* (B. crassifolia), *indoganhakusai* (B. purpurascens), and *chihakusai* (B. scopulosa); those belonging to the genus Chrysosplenium, such as *shirobananekonomesou* (C. album), *botannekonomesou* (C. kiotense), *kinshibebotannekonomesou* (C. kiotense forma xanthandrum), *nekonomesou* (C. grayanum), and *yamanekonomesou* (C. japonicum); those belonging to the genus Deutzia, such as crenate pride-of-Rochester (D. crenata), *sarasautsugi* (D. crenata f. plenta), Japanese snow flower (D. gracilis), and fuzzy pride-of-Rochester (D. seabra); those belonging to the genus Hydrangea, such as wild hydrangea (H. arborescens), *koazisai* (H. hirta), *kogakuutsugi* (H. luteovenosa), bigleaf hydrangea (H. macrophylla), PeeGee hydrangea (H. paniculata), Climing hydrangea (H. petiolaris), *gakuutsugi* (H.scandens), and mountain hydrangea (H. serrata); those belonging to the genus Mitella, such as *kochamerusou* (M. pauciflora); those belonging to the genus Parnassia, such as *shirahigesou* (P. foliosa var. nummularia), grass of Parnassus (P. palustris var. multiseta), and *ezoumebathisou* (P. palustris var. palustris); those belonging to the genus Penthorum, such as *takonoashi* (P. chinense); those belonging to the genus Philadelphus, such as Satsuma mock orange (P. satsumi); those belonging to the genus Ribes, such as *yashabishaku* (R. ambiguum), *yabusanzashi* (R. fasciculatum), and *komagatakesuguri* (R. japonicum); those belonging to the genus Rodgersia, such as *yagurumasou* (R. podophylla); those belonging to the genus Saxifraga, such as yellow saxifrage (S. aizoides), rough saxifrage (S. aspera), *nameradaimonjisou* (S. fortunei var. suwoensis), starry saxifrage (S. stellaris), and creeping saxifrage (S. stolonifera); and those belonging to the genus Schixophragma, such as Japanese hydrangea vine (S. hydrangeoides).

Among these plants belonging to the family Saxifragaceae, preferred are those belonging to the genus Bergenia or Saxifraga. The roots and stems thereof are mainly used.

Examples of the seaweeds include those belonging to the family Ulvaceae, for example, those belonging to the genus Ulva, Enteromorpha, or Ulvaria, and preferred are those belonging to the genus Ulva or Enteromorpha. Examples of seaweeds belonging to the family Gracilariaceae used in the present invention include those belonging to the genus Gracilaria, Gracilariopsis, Tylotus, or Gelidiopsis, and preferred are those belonging to the genus Gracilaria or Gracilariopsis. Examples of seaweeds belonging to the family Gelidiaceae used in the present invention include those belonging to the genus Gelidium, Beckerella, Pterocladia, or Acanthopeltis, and preferred are those belonging to the genus Gelidium. Examples of seaweeds belonging to the family Solieriaceae used in the present invention include those belonging to the genus Eucheuma, Meristotheca, Solieria, or Turnerella, and preferred are those belonging to the genus Eucheuma or Meristotheca. Examples of seaweeds belonging to the family Laminariaceae used in the present invention include those belonging to the genus Laminaria, Kjellmaniella, Ecklonia, Eisenia, Eckloniopsis, Agarum, Costaria, Arthrothamnus, or Hedophyllum, and preferred are those belonging to the genus Laminaria, Kjellmaniella, Ecklonia, Eisenia, or Eckloniopsis. Examples of seaweeds belonging to the family Alariaceae used in the present invention include those belonging to the genus Undaria or Alaria. Examples of seaweeds belonging to the family Sargassaceae used in the present invention include those belonging to the genus Hizikia, Sargassum, Turbinaria, Cystoseira, Myagropsis, or Coccophora, and preferred are those belonging to the genus Hizikia or Sargassum. Examples of seaweeds belonging to the family Fucaceae used in the present invention include those belonging to the genus Ascophyllum, Fucus, or Pelvetia, and preferred are those belonging to the genus Ascophyllum or Fucus. Examples of seaweeds belonging to the family Gloiopeltidaceae used in the present invention include those belonging to the genus Gloiopeltis. Examples of seaweeds belonging to the family Monostromataceae used in the present invention include those belonging to the genus Monostroma or Kornmannia, and preferred are those belonging to the genus Monostroma. Examples of seaweeds belonging to the family Codiaceae used in the present invention include those belonging to the genus Codium. Examples of seaweeds belonging to the family Bangiaceae used in the present invention include those belonging to the genus Porphyra or Bangia, and preferred are those belonging to the genus Porphyra. Examples of seaweeds belonging to the family Gigartinaceae used in the present invention include those belonging to the genus Gingartina, Rhodoglossum, or Chondrus, and preferred are those belonging to the genus Gingartina or Chondrus. Examples of seaweeds belonging to the family Bonnemaisoniaceae used in the present invention include those belonging to the genus Asparagopsis or Bonnemaisonia, and preferred are those belonging to the genus Asparagopsis. Examples of seaweeds belonging to the family Hypneaceae used in the present invention include those belonging to the genus Hypnea. Examples of seaweeds belonging to the family Chordariaceae used in the present invention include those belonging to the genus Analipus or Cladosiphon. Examples of seaweeds belonging to the family Nemacystaceae used in the present invention include those belonging to the genus Nemacystis. Examples of seaweeds belonging to the family Durvilleaceae used in the present invention include those belonging to the genus Durvillea. Examples of seaweeds belonging to the family Lessoniaceae used in the present invention include those belonging to the genus Lessonia or Macrocystis. Examples of seaweeds belonging to the family Rhodymeniaceae used in the present invention include those belonging to the genus Rhodymenia.

These plant extracts can be used either alone or in combination with two or more. The content of the plant extract used in the present invention is preferably from 0.0001 to 5% by mass, more preferably from 0.002 to 3% by mass, and even more preferably from 0.003 to 1% by mass, in terms of solid content based on the total amount of the composition. A content in the range can sufficiently exhibit the effect of the plant extract by the synergistic effect with the occluding properties of the composition containing components (A) and (B) and is therefore preferable.

As the skin-activating component (D) used in the present invention, examples of the anti-inflammatory agent include γ-amino-β-hydroxybutyric acid or salts thereof, γ-aminobutyric acid or salts thereof, L-carnitine or salts thereof, glycyrrhizic acid or salts thereof, glycyrrhetinic acid or salts thereof, isopropyl aminocaproic acid or salts thereof, allantoin, lysozyme chloride, guaiazulene, methyl salicylate, and γ-orizanol. In these agents, preferred are γ-amino-β-hydroxybutyric acid or salts thereof, γ-aminobutyric acid or salts thereof, L-carnitine and salts thereof, diisopropylamine dichloroacetate, glycyrrhizic acid or salts thereof, glycyrrhetinic acid or salts thereof, allantoin, lysozyme chloride, and methyl salicylate. Among them, preferred are γ-amino-β-hydroxybutyric acid or salts thereof, γ-aminobutyric acid or salts thereof, L-carnitine or salts thereof, diisopropylamine dichloroacetate, and glycyrrhizic acid or salts thereof.

The salts are not particularly limited, and examples thereof include salts of alkali metals such as sodium and potassium; inorganic salts of calcium, magnesium, aluminum, and zinc; and organic salts of organic amines such as ammonia, monoethanolamine, diethanolamine, and triethanolamine and of basic amino acids such as arginine and lysine. These salts can be used alone or as a mixture of two or more. From the viewpoint of easy availability in the market, salts of sodium, potassium, triethanolamine, and arginine are preferred.

The content of these anti-inflammatory agents is preferably from 0.001 to 5% by mass, more preferably from 0.01 to 2% by mass, and even more preferably from 0.01 to 1% by mass, based on the total amount of the composition. A content in the range can exhibit high effects of improving rough skin, inhibiting water evaporation, and occluding and can be easily blended into an aqueous phase and is therefore preferred.

As the skin-activating component (D) used in the present invention, examples of the moisturizing agent include N-methyl-L-serine, chondroitin sulfate or salts thereof, collagen (average molecular weight: 50000 to 6000000), water-soluble collagen, hydrolyzed collagen (average molecular weight: 100 to 9000), royal jelly extract, lactic acid or salts thereof, elastin, chitin, chitosan, urea, and pyrrolidonecarboxylic acid or salts thereof. In these agents, preferred are N-methyl-L-serine, chondroitin sulfate or salts thereof, water-soluble collagen, hydrolyzed collagen (average molecular weight: 100 to 9000), royal jelly extract, and lactic acid or salts thereof. Among them, preferred are N-methyl-L-serine, water-soluble collagen, and hydrolyzed collagen (average molecular weight: 100 to 9000).

The salts are not particularly limited, and examples thereof include salts of alkali metals such as sodium and potassium; inorganic salts of calcium, magnesium, aluminum, and zinc; and organic salts of organic amines such as ammonia, monoethanolamine, diethanolamine, and triethanolamine and of basic amino acids such as arginine and lysine. These salts can be used alone or as a mixture of two or more. From the viewpoint of easy availability in the market, salts of sodium, potassium, triethanolamine, and arginine are preferred.

The content of these anti-inflammatory agents is preferably from 0.001 to 20% by mass, more preferably from 0.01 to 15% by mass, and even more preferably from 0.01 to 10% by mass, based on the total amount of the composition. A content in the range can exhibit high moisturizing effect, water-evaporation inhibiting effect, and occluding effect and can be blended into an aqueous phase and is therefore preferred.

As the skin-activating component (D) used in the present invention, examples of the amino acid or salts thereof include ornithine, tryptophan, lysine, arginine, histidine, canavanine, glutamic acid, aspartic acid, serine, alanine, glycine, leucine, isoleucine, proline, threonine, valine, methionine, cystine, cysteine, hydroxyproline, phenylalanine, tyrosine, hydroxylysine, trimethylglycine, sodium aspartate, potassium aspartate, magnesium aspartate, calcium aspartate, sodium glutamate, potassium glutamate, magnesium glutamate, calcium glutamate, glutamic acid hydrochloride, cysteine hydrochloride, histidine hydrochloride, histidine acetate, histidine phosphate, lysine hydrochloride, lysine acetate, ornithine hydrochloride, ornithine acetate, tryptophan hydrochloride, arginine glutamate, ornithine glutamate, lysine glutamate, lysine aspartate, ornithine aspartate, and ε-aminocaproic acid. In these amino acids and salts thereof, preferred are arginine, lysine, hydroxylysine, histidine, or salts thereof. Arginine or salts thereof are even more preferred.

These amino acids and salts thereof can be used alone or in combination with two or more, and the content thereof is preferably from 0.0001 to 15% by mass, more preferably from 0.001 to 10% by mass, and even more preferably from 0.01 to 8% by mass, based on the total amount of the composition. A content in the range can exhibit more excellent effects of moisturizing, preventing or improving wrinkle formation, and preventing or improving rough skin and more excellent sense of use and stability and is therefore preferred.

As the skin-activating component (D) used in the present invention, any whitening agent that is usually used in cosmetics can be used without particular limitation, and examples thereof include L-ascorbic acid or derivatives thereof, rhododendrol or derivatives thereof, arbutin, tranexamic acid or derivatives thereof, and 4-methoxysalicylic acid or salts thereof.

Examples of ascorbic acid or derivatives thereof include, but not limited to, ascorbic acid or salts thereof, ascorbic acid ester or salts thereof, ascorbic acid phosphate or salts thereof, ascorbic acid sulfate or salts thereof, ascorbic acid glucoside (2-O-α-D-glucopyranosyl-L-ascorbic acid) or salts thereof, glucosamine ascorbate, and dehydroascorbic acid or salts thereof. In these derivatives, preferred are ascorbic acid or salts thereof, ascorbic acid ester or salts thereof, ascorbic acid phosphate or salts thereof, ascorbic acid sulfate or salts thereof, ascorbic acid-2-glucoside (2-O-α-D-glucopyranosyl-L-ascorbic acid) or salts thereof. Among them, preferred are ascorbic acid or salts thereof and ascorbic acid-2-glucoside (2-O-α-D-glucopyranosyl-L-ascorbic acid).

Examples of rhododendrol or derivatives thereof include, but not limited to, acylated rhododendrol, such as rhododendrol[4-(p-hydroxyphenyl)-2-butanol], acetyl rhododendrol, hexanoyl rhododendrol, octanoyl rhododendrol, dodecanoyl rhododendrol, tetradecanoyl rhododendrol, hexadecanoyl rhododendrol, octadecanoyl rhododendrol, 4-(3-acetoxybutyl)phenylacetate, 4-(3-propanoyloxybutyl)phenylpropanoate, 4-(3-octanoyloxybutyl)phenyloctanoate, and 4-(3-palmitoyloxybutyl)phenylpalmitate; alkyl ethers of rhododendrol, such as 4-(3-methoxybutyl)phenol, 4-(3-ethoxybutyl)phenol, and 4-(3-octyloxybutyl)phenol; and glycosides of rhododendrol, such as rhododendrol D-glucoside (α or β form), rhododendrol D-galactoside (α or β form), rhododendrol D-xyloside (α or β form), and rhododendrol D-maltoside (α or β form). In these Rhododendrol and derivatives thereof, preferred are rhododendrol and acylated rhododendrol. Among them, preferred is rhododendrol.

Examples of tranexamic acid or derivatives thereof include, but not limited to, tranexamic acid or salts thereof, hydrochloric acid trans-4-(trans-aminomethylcyclohexanecarbonyl)aminomethylcyclohexanecarb oxylic acid, trans-4- aminomethylcyclohexanecarboxylic acid 4'-hydroxyphenylester 2-(trans-4-aminomethylcyclohexylcarbonyloxy)-5-hydroxybenzoic acid or salts thereof, trans-4-aminomethylcyclohexanecarboxylic acid methylamide or salts thereof, trans-4-(P-methoxybenzoyl)aminomethylcyclohexanecarboxylic acid or salts thereof, and trans-4-guanidinomethylcyclohexanecarboxylic acid and salts thereof. In these tranexamic acid or derivatives thereof, preferred are tranexamic acid or salts thereof.

The salts are not particularly limited, and examples thereof include salts of alkali metals such as sodium and potassium; inorganic salts of calcium, magnesium, aluminum, and zinc; and organic salts of organic amines such as ammonia, monoethanolamine, diethanolamine, and triethanolamine and of basic amino acids such as arginine and lysine. These salts can be used alone or as a mixture of two or more. From the viewpoint of easy availability in the market, salts of sodium, potassium, triethanolamine, and arginine are preferred.

These whitening agents can be used alone or in combination with two or more. From the viewpoints of a whitening effect, emulsion stability, and sense of use, the content of the agents is preferably from 0.01 to 30% by mass, more preferably from 0.01 to 10% by mass, and even more preferably from 0.01 to 5% by mass, based on the total amount of the composition. A content in the range can give a sufficient whitening effect and excellent sense of use and stability and is therefore preferred.

As component (D) used in the present invention, examples of the singlet oxygen quencher or the antioxidant include carotenoids such as α-carotene, β-carotene, γ-carotene, lycopene, cryptoxanthin, lutein, zeaxanthin, isozeaxanthin, rhodoxanthin, capsanthin, and crocetin; 1,4-diazacyclooctane, 2,5-dimethylfuran, 2-methylfuran, 2,5-diphenylfuran, 1,3-diphenylisobenzofuran, α-tocopherol, β-tocopherol, γ-tocopherol, d-tocopherol, histidine, tryptophan, methionine, alanine, and alkyl esters thereof; tannins such as dibutylhydroxytoluene, butylhydroxyanisole, ascorbic acid, tannic acid, epicatechin, epigallocatechin, epicatechin gallate, and epigallocatechin gallate; and flavonoid such as lutin. In these singlet oxygen quenchers or antioxidants, preferred are carotene, ascorbic acid, tannic acid, epicatechin gallate, and epigallocatechin gallate.

These singlet oxygen quenchers or antioxidants can be used alone or in combination with two or more, and the content thereof is preferably from 0.001 to 5% by mass, more preferably from 0.01 to 2% by mass, and even more preferably from 0.01 to 1% by mass, based on the total amount of the composition. A content in the range can give a high effect of preventing or improving wrinkle formation and favorable sense of use and stability.

As the skin-activating component (D) used in the present invention, examples of the blood circulation accelerator include α-tocopherol, β-tocopherol, γ-tocopherol, d-tocopherol, nicotinamide, and methyl nicotinate. Among them, preferred are nicotinamide and methyl nicotinate.

These blood circulation accelerators can be used alone or in combination with two or more, and the content thereof is preferably from 0.001 to 10% by mass, more preferably from 0.01 to 5% by mass, and even more preferably from 0.05 to 3% by mass, based on the total amount of the composition. A content in the range enhances effects of preventing or improving deterioration in skin tension and elasticity and dullness of complexion, a moisturizing effect, an effect of preventing or improving rough skin, an effect of preventing or improving wrinkle formation, and an effect of preventing or improving spots and freckles, and gives excellent sense of use and stability, and is therefore preferred.

As the skin-activating component (D) used in the present invention, examples of the sebum secretion inhibitor include oxendolone, 17-α-methyl-β-nortestosterone, chlormadinone acetate, cyproterone acetate, spironolactone, hydroxyflutamide, estradiol, ethinylestradiol, zinc sulfocarbolate, zinc oxide, aluminum hydroxychloride, allantoin dihydroxyaluminum, vitamin B6, 13-cis-retinoic acid, vitamin E, glycyrrhetinic acid, salicylic acid, nicotinic acid, calcium pantothenate, dipotassium azelate, 10-hydroxyundecanoic acid, and 12-hydroxystearic acid. In these sebum secretion inhibitors, preferred are vitamin B6, glycyrrhetinic acid, salicylic acid, nicotinic acid, calcium pantothenate, and dipotassium azelate. Among them, preferred are vitamin B6, glycyrrhetinic acid, salicylic acid, and nicotinic acid.

These sebum secretion inhibitors can be used alone or in combination with two or more, and the content thereof is preferably from 0.01 to 10% by mass, more preferably from 0.1 to 5% by mass, based on the total amount of the composition. A content in the range enhances an effect of preventing or improving acne, an effect of inhibiting outstanding pores, and an effect of making the skin beautiful and give more excellent sense of use and stability and is therefore preferred.

As the skin-activating component (D) used in the present invention, examples of the antibacterial agent include sulfur, triclosan, trichlorocarbanilide, chlorhexine hydrochloride, benzalkonium chloride, benzethonium chloride, cetylpyridinium chloride, benzalkonium alkylphosphate, isopropyl methylphenol, benzoic acid, photosensitizer No. 201, and resorcin. Among them, preferred is isopropyl methylphenol. When these antibacterial agents are used, the content thereof is preferably from 0.0001 to 5% by mass, more preferably from 0.001 to 2% by mass, and even more preferably from 0.01 to 1% by mass, based on the total amount of the composition.

As the skin-activating component (D) used in the present invention, examples of the keratolytic agent include salicylic acid, N-acetylcysteine, lactic acid, citric acid, succinic acid, malic acid, alkylamine oxide, and benzoyl peroxide. Among them, preferred are salicylic acid and citric acid. When these keratolytic agents are used, the content thereof is preferably from 0.01 to 10% by mass, more preferably from 0.1 to 1% by mass, based on the total amount of the composition.

The aqueous composition as used according to the present invention can contain, in addition to the above-mentioned essential components, higher alcohols, fatty acids, esters, sterols, sterol-fatty acid esters, hydrocarbons, fats and oils, silicone oils, oxidation inhibitors, antifungal antiseptic agents, insect repellents, salts, chelating agents, neutralizers, pH adjusters, flavors, etc., within a range that does not impair the purpose of the present invention.

The formulation of the aqueous composition as used according to the present invention is not particularly limited as long as the aqueous phase is the continuous phase and can be, for example, emulsion, gel, spray, or mousse.

The use of the aqueous composition according to the present invention includes cosmetics, drugs and foods (including health foods). In the case of containing the skin-activating component (D), it is preferable to use the aqueous composition as a composition for external use on skin. Use of such a composition for external use on skin is not particularly limited, and the composition for external use on skin can be suitably used in cosmetics and drugs. Specifically, the composition for external use on skin can be suitably used in hair cosmetics such as shampoo, rinse, and conditioners; skin cosmetics such as facial cleansers, cleansing cosmetics, lotions, milky lotions, beauty creams, base cosmetics, sunscreen cosmetics, facial packs, and massage cosmetics; ointments containing various drugs; and external medicines such as creams. In particular, the composition for external use on skin of the present invention has a high water-occluding effect, and it is therefore preferable to use it as a composition for external use on skin that is kept on the skin without being washed out. Thus, the composition for external use on skin is suitable for use as cosmetics for moisture coating having good sense of use.

The container used in the present invention has a certain degree of sealability and is not particularly limited as long as it can be suitably used for the above-mentioned uses, including bottles with lids, jar bottles, tubes, spray containers, pump containers, and pump foamer containers. These containers filled with the aqueous composition can be used.

In particular, when a discharge container, such as a spray container, pump container, or pump foamer container, filled with the aqueous composition according to the present invention is used, even if the composition contains solid lipid or powder that tends to readily solidify, deterioration (change in color and/or odor) and clogging at the discharge opening can be prevented. Thus, the aqueous composition can be suitably used.

### [Examples]

The present invention will now be more specifically described by examples and comparative examples, but is not limited to the following examples. Note that the amount of each component is expressed in % by mass.

### Test 1: Water-evaporation inhibiting effect of polyoxyethylene alkyl ether

Five grams of each of samples 1 to 15 having components shown in Table 1 were placed in respective dishes having an opening area of 13.85 cm² and were left at a humidity of 30% and a temperature of 30°C. Changes in weight of each sample were measured 1, 3, 5, 7, 12, 18, and 24 hr later. The slope of the changes in weight, the n of nX+m (X represents time (hr)) calculated by a least-squares method, was defined as the water evaporation rate (unit: mg/h), and the absolute value thereof was plotted. The test was conducted three times for each sample, and the average value thereof was determined. Note that a lower water evaporation rate means higher inhibition of evaporation.

**[Table 1]**

| (Component) | Content (%) |
|---|---|
| POE alkyl ether shown in Table 2 | 1 |
| 2% Carboxyvinyl polymer (Synthalen K, manufactured by Wako Pure Chemical Industries, Ltd.) | 5 |
| 10% Potassium hydroxide | 0.37 |
| Methyl parahydroxybenzoate | 0.1 |
| Pure water | balance |

**[Table 2]**

| | POE alkyl ether | | Average molar number of ethylene oxide added | Water evaporation rate (mg/h) |
|---|---|---|---|---|
| | Alkyl group | Carbon number | | |
| Sample 1 | Lauryl | C12 | 2 | 134 |
| Sample 2 | Cetyl | C16 | 2 | 88 |
| Sample 3 | Stearyl | C18 | 2 | 80 |
| Sample 4 | Oleyl | C18 | 2 | 128 |
| Sample 5 | Behenyl | C22 | 2 | 41 |
| Sample 6 | Mixture | C12 to 15 | 2 | 118 |
| Sample 7 | Isostearyl | C18 | 2 | 125 |
| Sample 8 | Lauryl | C12 | 4 | 121 |
| Sample 9 | Cetyl | C16 | 5.5 | 116 |
| Sample 10 | Stearyl | C18 | 4 | 77 |
| Sample 11 | Oleyl | C18 | 4 | 122 |
| Sample 12 | Behenyl | C22 | 5 | 120 |
| Sample 13 | Mixture | C12 to 15 | 4 | 125 |
| Sample 14 | Isostearyl | C18 | 4 | 123 |

Test 1 reveals that polyoxyethylene behenyl ether having an average molar number of ethylene oxide added of 1.5 to 4, in particular, 2 shows an excellent water inhibiting effect, compared with other polyoxyethylene alkyl ethers including polyoxyethylene behenyl ether having an average molar number of ethylene oxide added of 5.

### Test 2: Concentration dependency on the effect of inhibiting water evaporation of polyoxyethylene alkyl ether

Changes in weight of samples 15 to 25 shown in Table 3 were measured under the same conditions as in Test 1, and water evaporation rates were determined. In test samples 24 and 25, precipitation of the polyoxyethylene behenyl ether was observed, and, therefore, the test for water evaporation rate was not conducted.

**[Table 3]**

| Component | | Sample 15 | Sample 16 | Sample 17 | Sample 18 | Sample 19 | Sample 20 | Sample 21 | Sample 22 | Sample 23 | Sample 24 | Sample 25 |
|---|---|---|---|---|---|---|---|---|---|---|---|---|
| 1 | Polyoxyethylene(2) behenyl ether | 0.01 | 0.1 | 0.2 | 0.5 | 1 | 2 | 5 | 10 | - | 0.1 | 1 |
| 2 | Carboxyvinyl polymer *1 | 0.1 | 0.1 | 0.1 | 0.1 | 0.1 | 0.1 | 0.1 | 0.1 | 0.1 | - | - |
| 3 | 10% Potassium hydroxide | 0.37 | 0.37 | 0.37 | 0.37 | 0.37 | 0.37 | 0.37 | 0.37 | 0.37 | - | - |
| 4 | Dipropylene glycol | 5 | 5 | 5 | 5 | 5 | 5 | 5 | 5 | 5 | 5 | 5 |
| 5 | Methyl parahydroxybenzoate | 0.1 | 0.1 | 0.1 | 0.1 | 0.1 | 0.1 | 0.1 | 0.1 | 0.1 | 0.1 | 0.1 |
| 6 | Pure water | balance | balance | balance | balance | balance | balance | balance | balance | balance | balance | balance |
| | Water evaporation rate (mg/h) | 120 | 57 | 40 | 40 | 40 | 40 | 40 | 40 | 121 | - | - |
| | Presence of precipitation of POE behenyl ether | No | No | No | No | No | No | No | No | - | Yes | Yes |

| | | | | | | | | | | | | |
|---|---|---|---|---|---|---|---|---|---|---|---|---|
| *1: Synthalen K (manufactured by Wako Pure Chemical Industries, Ltd.) | | | | | | | | | | | | |

Test 2 reveals that the polyoxyethylene behenyl ether shows a water-evaporation inhibiting effect in an amount of 0.1% by mass or more and shows a high water-evaporation inhibiting effect in an amount of 0.2% by mass or more.

### Test 3: Dependency of water-evaporation inhibiting effect by water-soluble polymer of polyoxyethylene alkyl ether

Changes in weight of samples of Examples 1 to 7 and Comparative Examples 1 to 7 having components shown below were measured under the same conditions as in Test 1, and water evaporation rates were determined. In addition, viscosities at 25°C were measured with a B-type viscometer (Vismetron viscometer: model VS-A1, Rotor No. 1, 12 rotations, 30 sec).

**[Table 4]**

| | Component | Example 1 | Example 2 | Example 3 | Example 4 | Example 5 | Example 6 | Example 7 |
|---|---|---|---|---|---|---|---|---|
| 1 | Polyoxyethylene(2) behenyl ether | 1 | 1 | 1 | 1 | 1 | 1 | 1 |
| 2 | Carboxyvinyl polymer *1 | 0.06 | - | - | - | - | - | - |
| 3 | Alkyl acrylate/methacrylate copolymer *2 | - | 0.06 | - | - | - | - | - |
| 4 | Xanthan gum *3 | - | - | 0.15 | - | - | - | - |
| 5 | Hydroxypropyl methylcellulose *4 | - | - | - | 0.3 | - | - | - |
| 6 | Polyvinyl alcohol *5 | - | - | - | - | 3 | - | - |
| 7 | Polyacrylamide *6 | - | - | - | - | - | 0.15 | - |
| 8 | (Sodium acrylate/sodium acryloyl dimethyl taurate) copolymer *7 | - | - | - | - | - | - | 0.15 |
| 9 | 10% Potassium hydroxide | 0.24 | 0.24 | - | - | - | - | - |
| 10 | Dipropylene glycol | 5 | 5 | 5 | 5 | 5 | 5 | 5 |
| 11 | Methyl parahydroxybenzoate | 0.1 | 0.1 | 0.1 | 0.1 | 0.1 | 0.1 | 0.1 |
| 12 | Pure water | balance | balance | balance | balance | balance | balance | balance |
| | Water evaporation rate (mg/h) | 42 | 41 | 43 | 45 | 51 | 49 | 45 |
| | Viscosity conditions: R-1-12 (mPa·s) | 138 | 120 | 212 | 105 | 188 | 156 | 100 |

**[Table 5]**

| Component | | Comparative Example 1 | Comparative Example 2 | Comparative Example 3 | Comparative Example 4 | Comparative Example 5 | Comparative Example 6 | Comparative Example 7 |
|---|---|---|---|---|---|---|---|---|
| 1 | Polyoxyethylene(2) behenyl ether | - | - | - | - | - | - | - |
| 2 | Carboxyvinyl polymer *1 | 0.06 | - | - | - | - | - | - |
| 3 | Alkyl acrylate/methacrylate copolymer *2 | - | 0.06 | - | - | - | - | - |
| 4 | Xanthan gum *3 | - | - | 0.15 | - | - | - | - |
| 5 | Hydroxypropyl methylcellulose *4 | - | - | - | 0.3 | - | - | - |
| 6 | Polyvinyl alcohol *5 | - | - | - | - | 3 | - | - |
| 7 | Polyacrylamide *6 | - | - | - | - | - | 0.15 | - |
| 8 | (Sodium acrylate/sodium acryloyl dimethyl taurate) copolymer *7 | - | - | - | - | - | - | 0.15 |
| 9 | 10% Potassium hydroxide | 0.24 | 0.24 | - | - | - | - | - |
| 10 | Dipropylene glycol | 5 | 5 | 5 | 5 | 5 | 5 | 5 |
| 11 | Methyl parahydroxybenzoate | 0.1 | 0.1 | 0.1 | 0.1 | 0.1 | 0.1 | 0.1 |
| 12 | Pure water | balance | balance | balance | balance | balance | balance | balance |
| | Water evaporation rate (mg/h) | 121 | 120 | 122 | 120 | 121 | 123 | 119 |
| | Viscosity conditions: R-1-12 (mPa·s) | 5 | 5 | 96 | 5 | 5 | 7 | 5 |

| | | | | | | | | |
|---|---|---|---|---|---|---|---|---|
| *1: Synthalen K (manufactured by Wako Pure Chemical Industries, Ltd.) *2: PEMULEN TR-1 (manufactured by Lubirizol Advanced Materials, Inc.) *3: KELTROL (manufactured by CP-Kelco) *4: Metolose 60 SH 10000 (manufactured by Shin-Etsu Chemical Co., Ltd.) *5: PVAEG-25 (manufactured by The Nippon Synthetic Chemical Industry Co., Ltd.) *6: SEPIGEL 501 (manufactured by SEPPIC, Inc.) *7: SIMULGEL EG (manufactured by SEPPIC, Inc.) | | | | | | | | |

### Manufacturing process

Component (1) and component (10) are uniformly mixed to prepare component A. Components (2) to (9), (11), and (12) are gradually added to component A, and the mixture is uniformly mixed to prepare component B.

Test 3 reveals that a combination of the polyoxyethylene behenyl ether and a water-soluble polymer shows a high water-evaporation inhibiting effect regardless of the type of the water-soluble polymer.

### Test 4: Evaluation of discharging performance in pump container

Pump containers were filled with the respective samples having components of Example 8 and Comparative Example 8 shown below, and the contents were discharged once. Subsequently, the pump containers were stored in a thermostat chamber at 25°C for 30 days and were then subjected to evaluation for discharging properties and long-term stability.

### Evaluation criteria

(1) Discharging property of discharge residue
   A: contents can be discharged without clogging of the contents
   B: contents are hard to be discharged because of slight clogging of the contents
   C: contents cannot be discharged because of clogging of the contents
(2) Long-term stability of discharge residue
   A: no separation and no change in color and odor of the contents occur
   B: slight separation or change in color or odor of the contents occur
   C: separation or change in color or odor of the contents occur

**[Table 6]**

| Component | Example 8 | Comparative Example 8 |
|---|---|---|
| Polyoxyethylene(2) behenyl ether | 1 | - |
| Glycerine | 10 | 10 |
| Dipropylene glycol | 10 | 10 |
| Polyethylene glycol 4000 | 5 | 5 |
| Squalane | 7 | 7 |
| 1,3-Butylene glycol | 2 | 2 |
| (Caprylic/capric/myristic/stearic)trigl yceryl | 1 | 1 |
| PEG-60 hydrogenated castor oil | 1 | 1 |
| Carboxyvinyl polymer *1 | 0.4 | 0.4 |
| Xanthan gum *3 | 0.2 | 0.2 |
| Sodium hyaluronate | 0.1 | 0.1 |
| 10% Potassium hydroxide | 2.0 | 2.0 |
| Edetate | 0.03 | 0.03 |
| Phenoxyethanol | 0.35 | 0.35 |
| Red No. 504 | trace quantity | trace quantity |
| Water | balance | balance |
| Discharging property | A | B |
| Stability over time | A | C |

| | | |
|---|---|---|
| *1: Synthalen K (manufactured by Wako Pure Chemical Industries, Ltd.) *3: KELTROL (manufactured by CP-Kelco) | | |

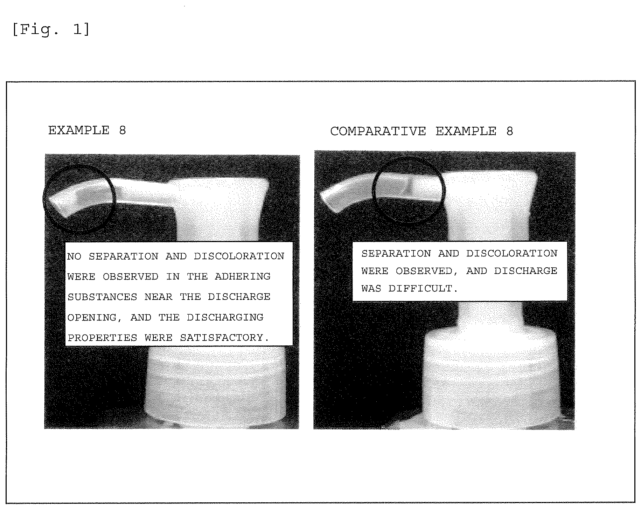

Test 4 reveals that the composition contained in a container of the present invention has satisfactory discharging properties and shows excellent long-term stability. Figure 1 shows the states of the pumps of Example 8 and Comparative Example 8, 30 days later. In Comparative Example 8, separation and discoloration of oil were observed, and discharge was difficult. In contrast, in Example 8, though the pharmaceutical preparation was partially drawn due to the negative pressure of the pump, no separation and discoloration were observed in the adhering substances near the discharge opening, and the discharging properties were not affected.

Plant extracts used in the following Examples will be described.

### Pyracantha fortuneana extract

The Pyracantha fortuneana extract is an extract of the fruit of Pyracantha fortuneana belonging to the genus Pyracantha in the family Rosaceae. The Pyracantha fortuneana extract was prepared as follows: 5.00 g of dried fruit of Pyracantha fortuneana was immersed in 50 mL of hot water of 90°C and boiled for 3 hr; the resulting extract was loaded on Daiaion HP-20 (manufactured by Mitsubishi Chemical Corp.) column (φ 3 cm x 11 cm, Vt = 80 mL), washed with 800 mL of an aqueous solution of 10% ethanol, subsequently eluted with 400 mL of an aqueous solution of 40 % ethanol; the eluate was concentrated under reduced pressure and lyophilized to give 1.02 g of a solid. To this solid, 17.34 g of glycerine and 2.04 g of purified water were added to give an extract having a solid content of 5%.

### Bergenia crassifolia L. extract

The Bergenia crassifolia L. extract is an extract of the rhizome of Bergenia crassifolia L. belonging to the genus Bergenia in the family Saxifragaceae. The Bergenia crassifolia L. was prepared as follows: 50 g of rhizome of Bergenia crassifolia L. was immersed in 250 mL of warm water at a temperature of 40 to 50°C and separated by filtration; the residue was similarly immersed in warm water several times to give 1.5 L of an extract; the extract was concentrated under reduced pressure; 100 mL of purified water was added to the residue; maturing was performed for 1 week; insoluble matter was removed by filtration; the extract was concentrated under reduced pressure again; and 1,3-butylene glycol was added thereto to adjust for giving an extraction solution having a solid content of 1% by mass.

### Magnolia obovata extract

The Magnolia obovata extract is an extract of bark (magnolia bark) of M. obovata belonging to the genus Magnolia in the family Magnoliaceae. As the Magnolia obovata extract, Magnolia obovata liquid K (solid content: 0.175%) manufactured by Ichimaru Pharcos Co., Ltd. was used.

### Citrus aurantium extract

The Citrus aurantium extract is an extract of a pericarp (orange peel) of Citrus aurantium belonging to the genus Citrus in the family Rutaceae. As the Citrus aurantium extract, orange peel liquid B (solid content: 2%) manufactured by Ichimaru Pharcos Co., Ltd. was used.

### C. lacryma-jobi var. ma-yuen extract

The C. lacryma-jobi var. ma-yuen extract is an extract of a seed (coix seed) excluding the seed coat of C. lacryma-jobi var. ma-yuen belonging to the genus Coix in the family Poaceae. As the C. lacryma-jobi var. ma-yuen extract, coix seed extract BG-S (solid content: 0.3%) manufactured by Maruzen Pharmaceuticals Co., Ltd. was used.

### Wheat germ extract

The wheat germ extract is an extract of the germ of wheat belonging to the genus Triticum in the family Poaceae. As the wheat germ extract, Clariskin (solid content: 11.7%) manufactured by Silab was used.

### Soybean extract

The soybean extract is a lactic acid bacteria fermentation product of soybean (soy milk) belonging to the genus Glycine in the family Leguminosae. As the soybean extract, a soy milk fermentation liquid (solid content: 2%) manufactured by Sansei Pharmaceutical Co., Ltd. was used.

### Hypericum erectum extract

The Hypericum erectum extract is an extract of H. erectum belonging to the genus Hypericum in the family Clusiaceae. As the Hypericum erectum extract, Pharcolex, Hypericum erectum B (solid content: 0.85%) manufactured by Ichimaru Pharcos Co., Ltd. was used.

### Rehmannia root extract

The Rehmannia root extract is an extract of the root of R. glutinosa Liboschitz Var. purpurea Makino or R. glutinosa Liboschitz belonging to the genus Rehmannia in the family Scrophulariaceae. As the Rehmannia root, a Rehmannia root extract, BG-J (solid content: 3%) was used.

### A. speciosa (Wendl.) K. Schum. extract

The A. speciosa (Wendl.) K. Schum. extract is an extract of the leaf of A. speciosa (Wendl.) K. Schum. belonging to the genus Alpinia in the family Zingiberaceae. As the A. speciosa (Wendl.) K. Schum. extract, an A. speciosa (Wendl.) K. Schum. leaf extract, BG (solid content: 3%) manufactured by Maruzen Pharmaceuticals Co., Ltd. was used.

### Seaweed extract

The seaweed extract is an extract mixture of seaweeds belonging to the genus Fucus in the family Fucaceae, the genus Laminaria in the family Laminariaceae, and the genus Chondrus and Gingartina in the family Gigartinaceae. As the seaweed extract, a seaweed extract M (solid content: 5.9%) manufactured by Maruzen Pharmaceuticals Co., Ltd. was used.

### P. armeniaca 1. extract

The P. armeniaca 1. extract is an extract of the seed of P. armeniaca 1. (apricot kernel) belonging to the genus Prunus in the family Rosaceae. As the P. armeniaca 1. extract, an apricot kernel extract, LA (solid content: 1.7%) was used.

### L. japonica extract

The L. japonica extract is an extract of the flower bud of L. japonica belonging to the genus Lonicera in the family Caprifoliaceae. As the L. japonica extract, Lonicerae flos extract-J (solid content: 3.5%) manufactured by Maruzen Pharmaceuticals Co., Ltd. was used.

### Nasturtium officinale extract

The Nasturtium officinale extract is an extract of watercress belonging to the genus Nasturtiumin the family Brassicaceae. As the Nasturtium officinale extract, WaterCress-KB (solid content: 3%) manufactured by Silab was used.

### Test 5: Test for occluding effect

One hundred microliters of each of the pharmaceutical preparations of Example 9 and Comparative Examples 9 and 10 shown in Table 7 were applied to a hydrophilic membrane filter (MF-Millipore GSWP02500 manufactured by Merck Millipore Japan Corporation, pore diameter: 0.22 µm, used by cutting the filter so as to have a filter diameter of 15 mm) and sufficiently dried on a hot plate of 37°C to prepare a sample-dried membrane filter. The sample-dried membrane filter was put on a cell containing 5 g of pure water and was left at a humidity of 30% and a temperature of 30°C. Changes in weight of the sample were measured 1, 3, 5, 7, 12, 18, and 24 hr later. The slope of the changes in weight, the n of nX+m (X represents time (hr)) calculated by a least-squares method, was defined as the water evaporation rate (unit: mg/h), and the absolute value thereof was plotted. The test was conducted three times for each sample, and the average value was determined. Note that a lower water evaporation rate means higher inhibition of evaporation. The results of the test are shown in Table 7. Table 7 reveals that the water-evaporation inhibiting effect is also maintained in the case of using polyoxyethylene alkyl or alkenyl ether having an alkyl or alkenyl group having 20 to 24 carbon atoms and an average molar number of ethylene oxide added of 1.5 to 4, a water-soluble polymer, and a skin-activating component.

**[Table 7]**

| | Component | Example 9 | Comparative Example 9 | Comparative Example 10 | Control |
|---|---|---|---|---|---|
| 1 | Polyoxyethylene(2) behenyl ether | 1 | - | 1 | - |
| 2 | polyoxyethylene(5) behenyl ether | - | 1 | - | - |
| 3 | Polyoxyethylene glycol 4000 | 5 | 5 | 5 | 5 |
| 4 | Glycerine | 5 | 5 | 5 | 5 |
| 5 | POE (100) hydrogenated castor oil | 0.1 | 0.1 | 0.1 | 0.1 |
| 6 | Dipotassium glycyrrhizate | 0.1 | 0.1 | - | - |
| 7 | Carboxyvinyl polymer *1 | 0.44 | 0.44 | 0.44 | 0.44 |
| 8 | 10% Potassium hydroxide | 2 | 2 | 2 | 2 |
| 9 | Dipropylene glycol | 5 | 5 | 5 | 5 |
| 10 | Pure water | balance | balance | balance | balance |
| | | | | | |
| | Occluding effect (water evaporation rate: mg/h) | 8.7 | 10.3 | 8.8 | 14.1 |

| | | | | | |
|---|---|---|---|---|---|
| *1: Synthalen K, manufactured by Wako Pure Chemical Industries, Ltd. | | | | | |

### Test 6: Test of using both POE alkyl ether and skin-activating component

Changes in weight of each of the pharmaceutical preparations of Examples 10 to 37 and Comparative Examples 11 to 38 shown in Tables 8 to 12 were measured under the same conditions as in Test 5 to determine water evaporation rates. Five special panelists for each evaluation item actually used the pharmaceutical preparations and evaluated "sense of moisturizing after application", "skin tension on the following morning", or "no stickiness during application" according to the evaluation criteria shown below. The evaluation results are shown as average points.

The pharmaceutical preparations of Examples 24 to 34 and Comparative Examples 28 to 38 were also evaluated for continuous use of twice a day (morning and night) for 2 months. "Effect of preventing or improving rough skin", "effect of preventing or improving outstanding pores, acnes, etc.", and "effect of preventing or improving spots and freckles" were evaluated according to the evaluation criteria shown below. The evaluation results are shown as average points.

The results are collectively shown in Tables 8 to 12. Tables 8 to 12 reveal that the occluding effect, the sense of moisturizing, the skin tension, no stickiness, and other performances are synergistically enhanced by blending skin-activating component (D), in addition to component (A) and component (B).

### [Panelist evaluation criteria]

| Evaluation criteria | Points |
|---|---|
| A high effect is sensed | 5 |
| An effect is sensed | 4 |
| A low effect is sensed | 3 |
| A slight effect is sensed | 2 |
| No effect is sensed | 1 |

**[Table 8]**

| | Component | Example 10 | Example 11 | Example 12 | Example 13 | Example 14 | Example 15 | Example 16 | Example 17 | Example 18 |
|---|---|---|---|---|---|---|---|---|---|---|
| 1 | Polyoxyethylene(2) behenyl ether | 1 | 1 | 1 | 1 | 1 | 1 | 1 | 1 | 1 |
| 2 | Pyracantha fortuneana extract | 0.1 | | | - | - | - | - | - | - |
| 3 | Bergenia crassifolia L. extract | - | 0.1 | - | - | - | - | - | - | - |
| 4 | Magnolia obovata extract | - | - | 0.1 | - | - | - | - | - | - |
| 5 | Citrus aurantium(Orange peel) extract | - | - | - | 0.1 | - | - | - | - | - |
| 6 | Coix lacryma-jobi var. ma-yuen (coix seed) extract | - | - | - | - | 0.1 | - | - | - | - |
| 7 | Wheat (germ) extract | - | - | - | - | - | 0.1 | - | - | - |
| 8 | Hypericum erectum extract | - | - | - | - | - | - | 0.1 | - | - |
| 9 | Rehmannia root extract | - | - | - | - | - | - | - | 0.1 | - |
| 10 | Alpinia speciosa K. Schum extract | - | - | - | - | - | - | - | - | 0.1 |
| 11 | Seaweed extract | - | - | - | - | - | - | - | - | - |
| 12 | Prunus armeniaca L. (Apricot kernel) extract | - | - | - | - | - | - | - | - | - |
| 13 | Lonicera japonica (Lonicerae flos) extract | - | - | - | - | - | - | - | - | - |
| 14 | Soybeans (soymilk fermentation liquid) extract | - | - | - | - | - | - | - | - | - |
| 15 | Nasturtium officinale extract | - | - | - | - | - | - | - | - | - |
| 16 | Carboxyvinyl polymer *1 | 0.2 | 0.2 | 0.2 | 0.2 | 0.2 | 0.2 | 0.2 | 0.2 | 0.2 |
| 17 | 10% Potassium hydroxide | 1 | 1 | 1 | 1 | 1 | 1 | 1 | 1 | 1 |
| 18 | Dipropylene glycol | 5 | 5 | 5 | 5 | 5 | 5 | 5 | 5 | 5 |
| 19 | PEG-60 hydrogenated castor oil | 0.2 | 0.2 | 0.2 | 0.2 | 0.2 | 0.2 | 0.2 | 0.2 | 0.2 |
| 20 | Methyl parahydroxybenzoate | 0.1 | 0.1 | 0.1 | 0.1 | 0.1 | 0.1 | 0.1 | 0.1 | 0.1 |
| 21 | Pure water | balance | balance | balance | balance | balance | balance | balance | balance | balance |
| | | | | | | | | | | |
| | Occluding effect (water evaporation rate: mg/h) | 9.2 | 9.3 | 9.2 | 9.4 | 9.5 | 9.2 | 9.4 | 9.3 | 9.1 |
| | | | | | | | | | | |
| | Sense of moisturizing | 4.2 | 4.4 | 4.3 | 4.2 | 4.2 | 4.2 | 4 | 4.1 | 4.2 |
| | Skin tension on the following morning | 4.5 | 4.4 | 4.5 | 4.4 | 4.3 | 4.4 | 4.5 | 4.3 | 4.3 |
| | No stickiness during application | 3.1 | 3.2 | 3.1 | 3 | 3 | 3.1 | 3.3 | 3 | 3.2 |

| | | | | | | | | | | |
|---|---|---|---|---|---|---|---|---|---|---|
| *1: Synthalen K (manufactured by Wako Pure Chemical Industries, Ltd.) | | | | | | | | | | |

**[Table 9]**

| | Component | Example 19 | Example 20 | Example 21 | Example 22 | Example 23 | Example 24 | Example 25 | Example 26 |
|---|---|---|---|---|---|---|---|---|---|
| 1 | Polyoxyethylene(2) behenyl ether | 1 | 1 | 1 | 1 | 1 | 1 | 1 | 1 |
| 2 | Pyracantha fortuneana extract | - | - | - | - | - | 0.1 | - | - |
| 3 | Bergenia crassifolia L. extract | - | - | - | - | - | 0.1 | - | - |
| 4 | Magnolia obovata extract | - | - | - | - | - | - | - | 0.1 |
| 5 | Citrus aurantium (Orange peel) extract | - | - | - | - | - | - | - | - |
| 6 | Coix lacryma-jobi var. ma-yuen (coix seed) extract | - | - | - | - | - | - | - | - |
| 7 | Wheat (germ) extract | - | - | - | - | - | - | - | - |
| 8 | Hypericum erectum extract | - | - | - | - | - | - | - | - |
| 9 | Rehmannia root extract | - | - | - | - | - | - | 0.1 | - |
| 10 | Alpinia speciosa K. Schum extract | - | - | - | - | - | - | - | - |
| 11 | Seaweed extract | 0.1 | - | - | - | - | - | 0.1 | - |
| 12 | Prunus armeniaca L. (Apricot kernel) extract | - | 0.1 | - | - | - | - | - | 0.1 |
| 13 | Lonicera japonica (Lonicerae flos) extract | - | - | 0.1 | - | - | - | - | - |
| 14 | Soybeans (soymilk fermentation liquid) extract | - | - | - | 0.1 | - | - | - | 0.1 |
| 15 | Nasturtium officinale extract | - | - | - | - | 0.1 | - | - | - |
| 16 | Carboxyvinyl polymer *1 | 0.2 | 0.2 | 0.2 | 0.2 | 0.2 | 0.2 | 0.2 | 0.2 |
| 17 | 10% Potassium hydroxide | 1 | 1 | 1 | 1 | 1 | 1 | 1 | 1 |
| 18 | Dipropylene glycol | 5 | 5 | 5 | 5 | 5 | 5 | 5 | 5 |
| 19 | PEG-60 hydrogenated castor oil | 0.2 | 0.2 | 0.2 | 0.2 | 0.2 | 0.2 | 0.2 | 0.2 |
| 20 | Methyl parahydroxybenzoate | 0.1 | 0.1 | 0.1 | 0.1 | 0.1 | 0.1 | 0.1 | 0.1 |
| 21 | Pure water | balance | balance | balance | balance | balance | balance | balance | balance |
| | | | | | | | | | |
| | Occluding effect (water evaporation rate: mg/h) | 9.3 | 9.2 | 9.2 | 9.2 | 9.2 | 9.3 | 9.2 | 9.3 |
| | | | | | | | | | |
| | Sense of moisturizing | 4.1 | 4.3 | 4.2 | 4.3 | 4.1 | 4.4 | 4.4 | 4.5 |
| | Skin tension on the following morning | 4.4 | 4.2 | 4.3 | 4.4 | 4.3 | 4.4 | 4.3 | 4.5 |
| | No stickiness during application | 3 | 3.1 | 3.2 | 3.1 | 3.2 | 3 | 3.1 | 3.1 |

| | | | | | | | | | |
|---|---|---|---|---|---|---|---|---|---|
| *1: Synthalen K (manufactured by Wako Pure Chemical Industries, Ltd.) | | | | | | | | | |

**[Table 10]**

| | Component | Comparative Example 11 | Comparative Example 12 | Comparative Example 13 | Comparative Example 14 | Comparative Example 15 | Comparative Example 16 | Comparative Example 17 | Comparative Example 18 | Comparative Example 19 |
|---|---|---|---|---|---|---|---|---|---|---|
| 1 | Polyoxyethylene(2) behenyl ether | - | - | - | - | - | - | - | - | - |
| 2 | Pyracantha fortuneana extract | 0.1 | - | - | - | - | - | - | - | - |
| 3 | Bergenia crassifolia L. extract | - | 0.1 | - | - | - | - | - | - | - |
| 4 | Magnolia obovata extract | - | - | 0.1 | - | - | - | - | - | - |
| 5 | Citrus aurantium (Orange peel) extract | - | - | - | 0.1 | - | - | - | - | - |
| 6 | Coix lacryma-jobi var. ma-yuen (coix seed) extract | - | - | - | - | 0.1 | - | - | - | - |
| 7 | Wheat (germ) extract | - | - | - | - | - | 0.1 | - | - | - |
| 8 | Hypericum erectum extract | - | - | - | - | - | - | 0.1 | - | - |
| 9 | Rehmannia root extract | - | - | - | - | - | - | - | 0.1 | - |
| 10 | Alpinia speciosa K. Schum extract | - | - | - | - | - | - | - | - | 0.1 |
| 11 | Seaweed extract | - | - | - | - | - | - | - | - | - |
| 12 | Prunus armeniaca L. (Apricot kernel) extract | - | - | - | - | - | - | - | - | - |
| 13 | Lonicera japonica (Lonicerae flos) extract | - | - | - | - | - | - | - | - | - |
| 14 | Soybeans (soymilk fermentation liquid) extract | - | - | - | - | - | - | - | - | - |
| 15 | Nasturtium officinale extract | - | - | - | - | - | - | - | - | - |
| 16 | Carboxyvinyl polymer *1 | 0.2 | 0.2 | 0.2 | 0.2 | 0.2 | 0.2 | 0.2 | 0.2 | 0.2 |
| 17 | 10% Potassium hydroxide | 1 | 1 | 1 | 1 | 1 | 1 | 1 | 1 | 1 |
| 18 | Dipropylene glycol | 5 | 5 | 5 | 5 | 5 | 5 | 5 | 5 | 5 |
| 19 | PEG-60 hydrogenated castor oil | 0.2 | 0.2 | 0.2 | 0.2 | 0.2 | 0.2 | 0.2 | 0.2 | 0.2 |
| 20 | Methyl parahydroxybenzoate | 0.1 | 0.1 | 0.1 | 0.1 | 0.1 | 0.1 | 0.1 | 0.1 | 0.1 |
| 21 | Pure water | balance | balance | balance | balance | balance | balance | balance | balance | balance |
| | | | | | | | | | | |
| | Occluding effect (water evaporation rate: mg/h) | 14.2 | 14.9 | 14.6 | 14.3 | 14 | 14.9 | 14.2 | 14.4 | 14.3 |
| | | | | | | | | | | |
| | Sense of moisturizing | 2.6 | 2.8 | 2.6 | 2.4 | 2.6 | 2.6 | 2.8 | 2.6 | 2.5 |
| | Skin tension on the following morning | 2.8 | 2.6 | 2.4 | 2.5 | 2.6 | 2.4 | 2.7 | 2.5 | 2.6 |
| | No stickiness during application | 3.1 | 3.1 | 3 | 3 | 3 | 3 | 3.1 | 3 | 3.1 |

| | | | | | | | | | | |
|---|---|---|---|---|---|---|---|---|---|---|
| *1 Synthalen K (manufactured by Wako Pure Chemical Industries, Ltd.) | | | | | | | | | | |

**[Table 11]**

| | Component | Comparative Example 20 | Comparative Example 21 | Comparative Example 22 | Comparative Example 23 | Comparative Example 24 | Comparative Example 25 | Comparative Example 26 | Comparative Example 27 |
|---|---|---|---|---|---|---|---|---|---|
| 1 | Polyoxyethylene(2) behenyl ether | - | - | - | - | - | - | - | - |
| 2 | Pyracantha fortuneana extract | - | - | - | - | - | 0.1 | - | - |
| 3 | Bergenia crassifolia L. extract | - | - | - | - | - | 0.1 | - | - |
| 4 | Magnolia obovata extract | - | - | - | - | - | - | - | 0.1 |
| 5 | Citrus aurantium (Orange peel) extract | - | - | - | - | - | - | - | - |
| 6 | Coix lacryma-jobi var. ma-yuen (coix seed) extract | - | - | - | - | - | - | - | - |
| 7 | Wheat (germ) extract | - | - | - | - | - | - | - | - |
| 8 | Hypericum erectum extract | - | - | - | - | - | - | - | - |
| 9 | Rehmannia extract | - | - | - | - | - | - | 0.1 | - |
| 10 | Alpinia speciosa K. Schum extract | - | - | - | - | - | - | - | - |
| 11 | Seaweed extract | 0.1 | - | - | - | - | - | 0.1 | - |
| 12 | Prunus armeniaca L. (Apricot kernel) extract | - | 0.1 | - | - | - | - | - | 0.1 |
| 13 | Lonicera japonica (Lonicerae flos) extract | - | - | 0.1 | - | - | - | - | - |
| 14 | Soybeans (soymilk fermentation liquid) extract | - | - | - | 0.1 | - | - | - | 0.1 |
| 15 | Nasturtium officinale extract | - | - | - | - | 0.1 | - | - | - |
| 16 | Carboxyvinyl polymer *1 | 0.2 | 0.2 | 0.2 | 0.2 | 0.2 | 0.2 | 0.2 | 0.2 |
| 17 | 10% Potassium hydroxide | 1 | 1 | 1 | 1 | 1 | 1 | 1 | 1 |
| 18 | Dipropylene glycol | 5 | 5 | 5 | 5 | 5 | 5 | 5 | 5 |
| 19 | PEG-60 hydrogenated castor oil | 0.2 | 0.2 | 0.2 | 0.2 | 0.2 | 0.2 | 0.2 | 0.2 |
| 20 | Methyl parahydroxybenzoate | 0.1 | 0.1 | 0.1 | 0.1 | 0.1 | 0.1 | 0.1 | 0.1 |
| 21 | Pure water | balance | balance | balance | balance | balance | balance | balance | balance |
| | | | | | | | | | |
| | Occluding effect (water evaporation rate: mg/h) | 14.3 | 14.2 | 14.5 | 14.4 | 14.3 | 14.1 | 14.3 | 14.2 |
| | | | | | | | | | |
| | Sense of moisturizing | 2.6 | 2.4 | 2.5 | 2.4 | 2.6 | 2.8 | 2.7 | 2.8 |
| | Skin tension on the following morning | 2.7 | 2.5 | 2.6 | 2.4 | 2.5 | 2.8 | 2.7 | 2.7 |
| | No stickiness during application | 3.2 | 3.3 | 3.2 | 3.1 | 3.2 | 3.1 | 3 | 3 |

| | | | | | | | | | |
|---|---|---|---|---|---|---|---|---|---|
| *1 Synthalen K (manufactured by Wako Pure Chemical Industries, Ltd.) | | | | | | | | | |

**[Table 12]**

| | Component | Example 27 | Example 28 | Example 29 | Example 30 | Example 31 | Example 32 | Example 33 | Example 34 | Example 35 | Example 36 | Example 37 |
|---|---|---|---|---|---|---|---|---|---|---|---|---|
| 1 | Polyoxyethylene(2) behenyl ether | 1 | 1 | 1 | 1 | 1 | 1 | 1 | 1 | 1 | 1 | 1 |
| 2 | γ-Amino-β-hydroxybutyric acid | 0.5 | - | - | - | - | - | - | - | 0.5 | - | 0.5 |
| 3 | γ-Aminobutyric acid | - | 0.5 | - | - | - | - | - | - | - | - | - |
| 4 | L-Carnitine | - | - | 0.5 | - | - | - | - | - | - | 0.5 | - |
| 5 | Rhododendrol | - | - | - | 2 | - | - | - | - | - | 2 | - |
| 6 | N-Methyl-L-serine | - | - | - | - | 0.5 | - | - | - | - | - | - |
| 7 | Niacinamide | - | - | - | - | - | 1 | - | - | 1 | - | - |
| 8 | Ascorbic acid-2-glucoside | - | - | - | - | - | - | 2 | - | - | - | 2 |
| 9 | Isopropyl methylphenol | - | - | - | - | - | - | - | 0.1 | - | - | - |
| 10 | Carboxyvinyl polymer *1 | 0.2 | 0.2 | 0.2 | 0.2 | 0.2 | 0.2 | 0.2 | 0.2 | 0.2 | 0.2 | 0.2 |
| 11 | 10% Potassium hydroxide | 1 | 1 | 1 | 1 | 1 | 1 | 1 | 1 | 1 | 1 | 1 |
| 12 | Dipropylene glycol | 10 | 10 | 10 | 10 | 10 | 10 | 10 | 10 | 10 | 10 | 10 |
| 13 | PEG-60 hydrogenated castor oil | 0.2 | 0.2 | 0.2 | 0.2 | 0.2 | 0.2 | 0.2 | 0.2 | 0.2 | 0.2 | 0.2 |
| 14 | Methyl parahydroxybenzoate | 0.1 | 0.1 | 0.1 | 0.1 | 0.1 | 0.1 | 0.1 | 0.1 | 0.1 | 0.1 | 0.1 |
| 15 | Pure water | balance | balance | balance | balance | balance | balance | balance | balance | balance | balance | balance |
| | | | | | | | | | | | | |
| | Occluding effect (water evaporation rate: mg/h) | 9.4 | 9.6 | 9.5 | 9.5 | 9.2 | 9.3 | 9.3 | 9.2 | 9.5 | 9.3 | 9.4 |
| | Sense of use | | | | | | | | | | | |
| | Sense of moisturizing | 4.3 | 4.1 | 4.4 | 4 | 4.3 | 4.3 | 4 | 4 | 4.3 | 4.2 | 4.2 |
| | Skin tension on the following morning | 4.4 | 4.3 | 4.5 | 4.1 | 4.4 | 4.4 | 4 | 4.1 | 4.5 | 4.5 | 4.5 |
| | No stickiness during application | 3.5 | 3.4 | 3.1 | 2.8 | 3.1 | 3.1 | 2.8 | 3 | 3.1 | 3 | 2.8 |
| | Two-month continuous use test | | | | | | | | | | | |
| | Effect of preventing or improving rough skin | 4.2 | 4.1 | 4.4 | 3.8 | 4.1 | 4.2 | 3.7 | 3.8 | 4.4 | 4.5 | 4.3 |
| | Effect of preventing or improving outstanding pores, acnes, etc. | - | - | - | - | - | - | - | 4.3 | - | - | - |
| | Effect of preventing or improving spots and freckles | - | - | - | 4.4 | - | - | 4.1 | - | - | 4.4 | 4.1 |

| | | | | | | | | | | | | |
|---|---|---|---|---|---|---|---|---|---|---|---|---|
| *1: Synthalen K (manufactured by Wako Pure Chemical Industries, Ltd.) | | | | | | | | | | | | |

**[Table 13]**

| | Component | Comparative Example 28 | Comparative Example 29 | Comparative Example 30 | Comparative Example 31 | Comparative Example 32 | Comparative Example 33 | Comparative Example 34 | Comparative Example 35 | Comparative Example 36 | Comparative Example 37 | Comparative Example 38 |
|---|---|---|---|---|---|---|---|---|---|---|---|---|
| 1 | Polyoxyethylene(2) behenyl ether | - | - | - | - | - | - | - | - | - | - | - |
| 2 | γ-Amino-β-hydroxybutyric acid | 0.5 | - | - | - | - | - | - | - | 0.5 | - | 0.5 |
| 3 | γ-Aminobutyric acid | - | 0.5 | - | - | - | - | - | - | - | - | - |
| 4 | L-Carnitine | - | - | 0.5 | - | - | - | - | - | - | 0.5 | - |
| 5 | Rhododendrol | - | - | - | 2 | - | - | - | - | - | 2 | - |
| 6 | N-Methyl-L-serine | - | - | - | - | 0.5 | - | - | - | - | - | - |
| 7 | Niacinamide | - | - | - | - | - | 1 | - | - | 1 | - | - |
| 8 | Ascorbic acid-2-glucoside | - | - | - | - | - | - | 2 | - | - | - | 2 |
| 9 | Isopropyl methylphenol | - | - | - | - | - | - | - | 0.1 | - | - | - |
| 10 | Carboxyvinyl polymer *1 | 0.2 | 0.2 | 0.2 | 0.2 | 0.2 | 0.2 | 0.2 | 0.2 | 0.2 | 0.2 | 0.2 |
| 11 | 10% Potassium hydroxide | 1 | 1 | 1 | 1 | 1 | 1 | 1 | 1 | 1 | 1 | 1 |
| 12 | Dipropylene glycol | 10 | 10 | 10 | 10 | 10 | 10 | 10 | 10 | 10 | 10 | 10 |
| 13 | PEG-60 hydrogenated castor oil | 0.2 | 0.2 | 0.2 | 0.2 | 0.2 | 0.2 | 0.2 | 0.2 | 0.2 | 0.2 | 0.2 |
| 14 | Methyl parahydroxybenzoate | 0.1 | 0.1 | 0.1 | 0.1 | 0.1 | 0.1 | 0.1 | 0.1 | 0.1 | 0.1 | 0.1 |
| 15 | Pure water | balance | balance | balance | balance | balance | balance | balance | balance | balance | balance | balance |
| | | | | | | | | | | | | |
| | Occluding effect (water evaporation rate: mg/h) | 14.4 | 14.6 | 14.4 | 14.2 | 14.2 | 14.5 | 14.3 | 14.2 | 14.4 | 14.3 | 14.4 |
| | Sense of use | | | | | | | | | | | |
| | Sense of moisturizing | 2.6 | 2.7 | 2.4 | 2.5 | 2.8 | 2.7 | 2.6 | 2.7 | 2.6 | 2.3 | 2.4 |
| | Skin tension on the following morning | 2.6 | 2.5 | 2.7 | 2.5 | 2.6 | 2.4 | 2.6 | 2.4 | 2.8 | 2.4 | 2.6 |
| | No stickiness during application | 3.4 | 3.3 | 3 | 2.8 | 3 | 3 | 2.8 | 3.1 | 3 | 3 | 2.8 |
| | Two-month continuous use test | | | | | | | | | | | |
| | Effect of preventing or improving rough skin | 3.9 | 3.8 | 3.8 | 3.6 | 3.5 | 4 | 3.7 | 3.8 | 3.9 | 4 | 3.9 |
| | Effect of preventing or improving outstanding pores, acnes, etc. | - | - | - | - | - | - | - | 4.3 | - | - | - |
| | Effect of preventing or improving spots and freckles | - | - | - | 4 | - | - | 3.8 | - | - | 4.1 | 3.8 |

| | | | | | | | | | | | | |
|---|---|---|---|---|---|---|---|---|---|---|---|---|
| *1: Synthalen K (manufactured by Wako Pure Chemical Industries, Ltd.) | | | | | | | | | | | | |

### Test 7: Concentration dependency of occluding effect of POE alkyl ether

Changes in weight of each of the pharmaceutical preparations of Examples 38 to 43 and Comparative Examples 39 to 42 shown in Table 14 were measured under the same conditions as in Test 5 to determine water evaporation rates. Sensory evaluation for "sense of moisturizing after application", "skin tension on the following morning", and "no stickiness during application" were performed as in Test 6. The results are collectively shown in Table 14. Table 14 reveals that the occluding effect, the sense of moisturizing, the skin tension, and no stickiness are synergistically enhanced by blending the skin-activating component, in addition to component (A) and component (B). In Comparative Examples 41 and 42, precipitation of the POE alkyl ether was observed, and, therefore, the sensory evaluation was not conducted.

**[Table 14]**

| | Component | Example 38 | Example 39 | Example 40 | Example 41 | Example 42 | Example 43 | Comparative Example 39 | Comparative Example 40 | Comparative Example 41 | Comparative Example 42 |
|---|---|---|---|---|---|---|---|---|---|---|---|
| 1 | Polyoxyethylene(2) behenyl ether | 0.01 | 0.1 | 0.2 | 0.5 | 1 | 2 | - | - | 0.1 | 1 |
| 2 | Vaseline | -- | - | - | - | - | - | 1 | - | - | - |
| 3 | Carboxyvinyl polymer *1 | 0.2 | 0.2 | 0.2 | 0.2 | 0.2 | 0.2 | 0.2 | 0.2 | - | - |
| 4 | 10% Potassium hydroxide | 1 | 1 | 1 | 1 | 1 | 1 | 1 | 1 | - | - |
| 5 | Citrus aurantium (Orange peel) extract | 0.1 | 0.1 | 0.1 | 0.1 | 0.1 | 0.1 | 0.1 | 0.1 | 0.1 | 0.1 |
| 6 | Dipropylene glycol | 5 | 5 | 5 | 5 | 5 | 5 | 5 | 5 | 5 | 5 |
| 7 | PEG-60 hydrogenated castor oil | 0.2 | 0.2 | 0.2 | 0.2 | 0.2 | 0.2 | 0.2 | 0.2 | 0.2 | 0.2 |
| 8 | Methyl parahydroxybenzoate | 0.1 | 0.1 | 0.1 | 0.1 | 0.1 | 0.1 | 0.1 | 0.1 | 0.1 | 0.1 |
| 9 | Pure water | balance | balance | balance | balance | balance | balance | balance | balance | balance | balance |
| | | | | | | | | | | | |
| | Occluding effect (water evaporation rate) | 14 | 13.4 | 10.1 | 9.5 | 9.4 | 9.3 | 12.3 | 14.5 | precipitation | precipitation |
| | Sense of moisturizing | 2.8 | 3.1 | 3.3 | 4.2 | 4.4 | 4.3 | 3.1 | 2.7 | - | - |
| | Skin tension on the following morning | 2.7 | 3.2 | 3.5 | 4.4 | 4.5 | 4.5 | 2.8 | 2.6 | - | - |
| | No stickiness during application | 3.2 | 3.3 | 3.2 | 3.1 | 3 | 2.8 | 3 | 3.3 | - | - |

| | | | | | | | | | | | |
|---|---|---|---|---|---|---|---|---|---|---|---|
| *1: Synthalen K (manufactured by Wako Pure Chemical Industries, Ltd.) | | | | | | | | | | | |

### Test 8: Change in water content of horny layer and transepidermal water loss by layered application

Eight special panelists were each provided with a mark of 2x4 cm on the inside of the forearm and applied with commercially available three basic skin-care products (lotion, milky lotion, and cream) and then with 1.25 µL/cm² of a night pack cosmetic product (test sample, Table 15). As a control test, application of only three basic skin-care products and no application of any product were performed.

At the time before the application, 30 min after the application, 3 hr after the application, and 7 hr after the application, water contents of horny layer were measured with Skicon-200EX (manufactured by I.B.S. Co., Ltd.) and transepidermal water losses were measured with Tewameter MPA580 (manufactured by Courage & Khazaka Electric GmbH) (measurement conditions: 22±1°C, RH50%±5%).

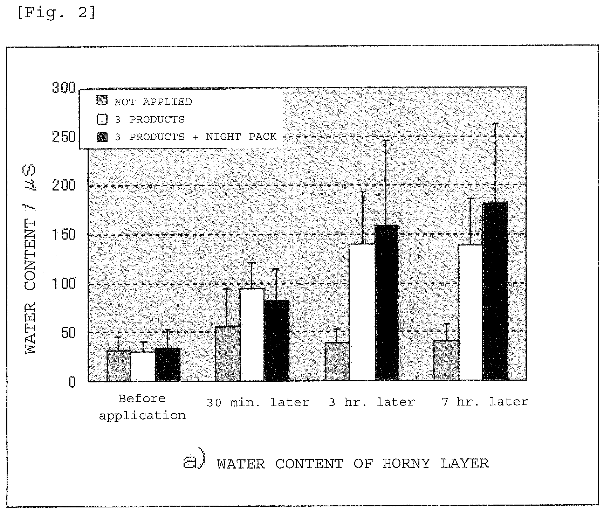

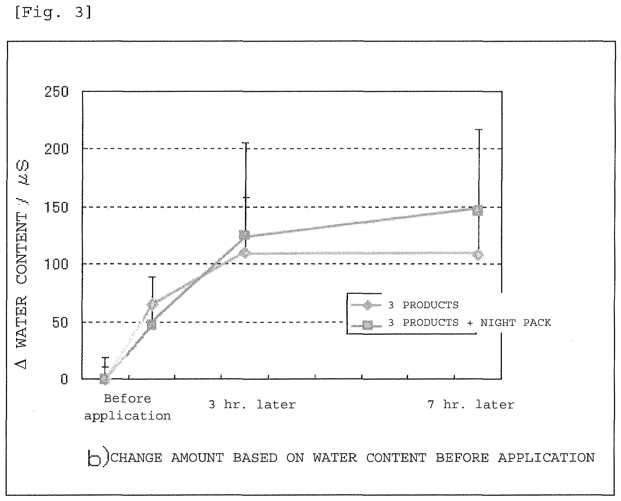

The respective results are shown in Figures 2 and 3. In Figures 2 and 3, NOT APPLIED shows the results when the basic skin-care products and the night pack cosmetic product were not applied; THREE PRODUCTS shows the results when only the three basic skin-care products were applied; THREE PRODUCTS+NIGHT PACK shows the results when the three basic skin-care products were applied and then the night pack cosmetic product was applied.

**[Table 15]**

| Component | % by mass |
|---|---|
| Polyoxyethylene(2) behenyl ether | 1 |
| Glycerine | 10 |
| Dipropylene glycol | 10 |
| Polyethylene glycol 4000 | 5 |
| Squalane | 7 |
| 1,3-Butylene glycol | 2 |
| (Caprylic/capric/myristic/stearic)triglyceryl | 1 |
| PEG-60 hydrogenated castor oil | 1 |
| Carboxyvinyl polymer *1 | 0.4 |
| Xanthan gum *2 | 0.2 |
| Sodium hyaluronate | 0.01 |
| 10% Potassium hydroxide | 2 |
| Glycine extract (manufactured by Sansei Pharmaceutical Co., Ltd.: soy milk fermentation liquid) | 0.1 |
| L. japonica extract (manufactured by Maruzen Pharmaceuticals Co., Ltd.: Lonicerae flos extract-J) | 0.1 |
| Nasturtium extract (manufactured by Silab: WaterCress-KB) | 0.1 |
| A. speciosa (Wendl.) K. Schum. extract (manufactured by Maruzen Pharmaceuticals Co., Ltd.: A. speciosa (Wendl.) K. Schum. leaf extract BG) | 0.1 |
| Seaweed extract (manufactured by Maruzen Pharmaceuticals Co., Ltd.: seaweed extract M) | 0.1 |
| Edetate | 0.03 |
| Phenoxyethanol | 0.35 |
| Water | balance |

| | |
|---|---|
| *1: Synthalen K (manufactured by Wako Pure Chemical Industries, Ltd.) *2: Keltrol (manufactured by CP Kelco) | |

It was revealed that when the night pack cosmetic product containing components (A), (B), and (D) of the present invention is applied after the skin care with usual three basic skin-care products (lotion, milky lotion, and cream), a high water content of horny layer can be maintained even 7 hr after the application.

### Test 9: Change in water content of horny layer in artificial rough skin

Rough skins were artificially formed on 10 special panelists according to the method of Kawashima, et al. (The Japanese Journal of Dermatology, 117(3), pp. 275-284, 2007) (i.e., a cup (diameter: 3.5 cm) was set on a flexor of the forearm, and 6 mL of a mixture of acetone and ether (with a mass ratio of 1:1) was added thereto. The solvents were removed 20 min later and then volatilized. Subsequently, 10 mL of distilled water was added thereto. The water was removed 5 min later. On the following day, the same portion was treated with the same procedure.)

The artificially roughened skin portion was marked, and an appropriate amount of the cream shown in Table 16 was applied to the portion twice a day (morning and night) for 6 days after the artificial formation of the rough skin.

On the day before and after (the 2nd day) the rough skin formation and on the 3rd day and the 6th day of using the cream, water contents of horny layer were measured with Skicon-200EX (manufactured by I.B.S. Co., Ltd.) (measurement conditions: 22±1°C, RH50%±5%). Difference in water content of horny layer on the 3rd or 6th day of using the cream from that after the rough skin formation is defined as a variation in water content of horny layer (ΔµS). The results are collectively shown in Table 16. A larger variation in water content of horny layer (ΔµS) means a higher improving effect.

**[Table 16]**

| | Component | Example 44 | Comparative Example 43 | Comparative Example 44 | Control |
|---|---|---|---|---|---|
| 1 | Polyoxyethylene(2) behenyl ether | 1 | - | 1 | - |
| 2 | γ-Amino-β-hydroxybutyric acid | 0.5 | 0.5 | - | - |
| 3 | Carboxyvinyl polymer *1) | 0.2 | 0.2 | 0.2 | 0.2 |
| 4 | Dipropylene glycol | 10 | 10 | 10 | 10 |
| 5 | PEG-60 hydrogenated castor oil | 0.5 | 0.5 | 0.5 | 0.5 |
| 6 | EDTA | 0.03 | 0.03 | 0.03 | 0.03 |
| 7 | 10% Potassium hydroxide | 0.92 | 0.92 | 0.92 | 0.92 |
| 8 | Phenoxyethanol | 0.35 | 0.35 | 0.35 | 0.35 |
| 9 | Water | 76.7 | 77.7 | 77.2 | 78.2 |
| | | | | | |
| | Variation in water content of horny layer (ΔµS) | | | | |
| | The 3rd day | 9.2 | 3.8 | 4.7 | 5.3 |
| | The 6th day | 31.4 | 26.6 | 26.6 | 25.7 |

| | | | | | |
|---|---|---|---|---|---|
| *1: Synthalen K (manufactured by Wako Pure Chemical Industries, Ltd.) | | | | | |

It is revealed that the water content of horny layer is synergistically improved by blending γ-amino-β-hydroxybutyric acid as component (D), in addition to component (A) and component (B). In particular, the effect of improving water content of horny layer was significantly enhanced by using component (A), compared with the pharmaceutical preparation containing component (D). The cause of this is assumed that in the state where epidermal lipids and water-soluble components were forcedly removed by the treatment of artificially roughening the skin, component (D) acts on the inside of the skin to form moisture inside the skin, and component (A) covers the epidermal surface and prevents the moisture from evaporating to lead the skin to good conditions.

Prescription examples of the composition for external use on skin of the present invention are shown below. Excellent occluding properties and a synergistic effect by the skin-activating component are expected in every composition.

**[Table 17]**

| Prescription Example 1 (lotion) | |
|---|---|
| Component | % by mass |
| Polyoxyethylene(2) behenyl ether | 1 |
| Dipropylene glycol | 10 |
| PEG-60 hydrogenated castor oil | 0.2 |
| Carboxyvinyl polymer *1 | 0.2 |
| Xanthan gum *2 | 0.2 |
| Sodium hyaluronate | 0.01 |
| 10% Potassium hydroxide | 1 |
| L-Carnitine (manufactured by Kongo Chemical Co., Ltd. | 0.5 |
| Rhododendrol (manufactured by Takasago International Corp.) | 2.0 |
| Isopropyl methylphenol (manufactured by Osaka Kasei Co., Ltd.: Biozole) | 0.1 |
| Pyracantha fortuneana extract (manufactured by Suntory Ltd.: Pyracantha fortuneana) | 0.1 |
| L. japonica extract (manufactured by Maruzen Pharmaceuticals Co., Ltd.: Lonicerae flos extract-J) | 0.1 |
| Nasturtium officinale extract (Manufactured by Silab: WaterCress-KB) | 0.1 |
| A. speciosa (Wendl.) K. Schum. extract (manufactured by Maruzen Pharmaceuticals Co., Ltd.: A. speciosa (Wendl.) K. Schum. leaf extract BG) | 0.1 |
| Phellodendron bark extract (manufactured by Maruzen Pharmaceuticals Co., Ltd.: Phellodendron extract J) | 0.1 |
| Apricot kernel extract (manufactured by Maruzen Pharmaceuticals Co., Ltd.: Apricot kernel LA) | |
| Edetate | 0.03 |
| Phenoxyethanol | 0.35 |
| Water | balance |

**[Table 18]**

| Prescription Example 2 (beauty essence) | |
|---|---|
| Component | % by mass |
| Polyoxyethylene(2) behenyl ether | 1 |
| 1,3-Butylene glycol | 10 |
| PEG-60 hydrogenated castor oil | 0.5 |
| Carboxyvinyl polymer *1 | 0.4 |
| Xanthan gum *2 | 0.2 |
| Sodium hyaluronate | 0.01 |
| 10% Potassium hydroxide | 2 |
| γ-Amino-β-hydroxybutyric acid (manufactured by Kaken Pharmaceutical Co., Ltd.: Bisamine) | 0.5 |
| Rhododendrol (manufactured by Takasago International Corp.) | 2.0 |
| N-Methyl-L-serine (manufactured by Takasago International Corp.) | 0.5 |
| Soybean extract (manufactured by Sansei Pharmaceutical Co., Ltd.: soy milk fermentation liquid) | 0.1 |
| L. japonica extract (manufactured by Maruzen Pharmaceuticals Co., Ltd.: Lonicerae flos extract-J) | 0.1 |
| Nasturtium officinale extract (manufactured by Silab: WaterCress-KB) | 0.1 |
| A. speciosa (Wendl.) K. Schum. extract (manufactured by Maruzen Pharmaceuticals Co., Ltd.: A. speciosa (Wendl.) K. Schum. leaf extract BG) | 0.1 |
| Seaweed extract (manufactured by Maruzen Pharmaceuticals Co., Ltd.: seaweed extract M) | 0.1 |
| Edetate | 0.03 |
| Phenoxyethanol | 0.35 |
| Water | balance |

**[Table 19]**

| Prescription Example 3 (milky lotion) | |
|---|---|
| Component | % by mass |
| Polyoxyethylene(2) behenyl ether | 1 |
| 1,3-Butylene glycol | 10 |
| PEG-60 hydrogenated castor oil | 1.0 |
| Squalane | 5.0 |
| Dimer dilinoleate (phytosterol/isostearyl/cetyl/stearyl/behenyl) (manufactured by Nippon Fine Chemical Co., Ltd.: Plandool-H) | 2.0 |
| Macadamia nut oil fatty acid phytosteryl (manufactured by Nippon Fine Chemical Co., Ltd.: YOFCO MAS) | 1.5 |
| Carboxyvinyl polymer *1 | 0.4 |
| Xanthan gum *2 | 0.2 |
| Sodium hyaluronate | 0.01 |
| 10% Potassium hydroxide | 2 |
| γ-Amino-β-hydroxybutyric acid (manufactured by Kaken Pharmaceutical Co., Ltd.: Bisamine) | 0.5 |
| Rhododendrol (manufactured by Takasago International Corp.) | 2.0 |
| Niacinamide (manufactured by Lonza Japan Ltd.) | 0.5 |
| Soybean extract (manufactured by Sansei Pharmaceutical Co., Ltd.: soy milk fermentation liquid) | 0.1 |
| L. japonica extract (manufactured by Maruzen Pharmaceuticals Co., Ltd.: Lonicerae flos extract-J) | 0.1 |
| Nasturtium officinale extract (manufactured by Silab: WaterCress-KB) | 0.1 |
| A. speciosa (Wendl.) K. Schum. extract | 0.1 |
| (manufactured by Maruzen Pharmaceuticals Co., Ltd.: A. speciosa (Wendl.) K. Schum. leaf extract BG) | |
| Seaweed extract (manufactured by Maruzen Pharmaceuticals Co., Ltd.: seaweed extract M) | 0.1 |
| Yeast extract (manufactured by Pentapharm Ltd.: Dismutin BTJ) | 0.1 |
| Edetate | 0.02 |
| Phenoxyethanol | 0.4 |
| Water | balance |

## Claims

1. A cosmetic, drug or food contained in a container, comprising an aqueous composition comprising the following components (A), (B), and (C):
(A) a polyoxyethylene alkyl or alkenyl ether having an alkyl or alkenyl group having 20 to 24 carbon atoms and an average molar number of ethylene oxide added of 1.5 to 4;
(B) a water-soluble polymer; and
(C) water.

2. The cosmetic, drug or food according to Claim 1, wherein component (A) is a polyoxyethylene alkyl or alkenyl ether having an alkyl or alkenyl group having 20 to 24 carbon atoms and an average molar number of ethylene oxide added of 1.5 to 2.5.

3. The cosmetic ,drug or food according to Claim 1 or 2, wherein the content of component (A) is from 0.05 to 20% by mass based on the total amount of the composition.

4. The cosmetic, drug or food according to any one of Claims 1 to 3, wherein component (B) is at least one selected from the group consisting of carboxyvinyl polymers, alkyl acrylate/methacrylate copolymers, xanthan gum, hydroxypropyl methylcellulose, polyacrylamides, and (sodium acrylate/sodium acryloyl dimethyl taurate) copolymers.

5. The cosmetic, drug or food according to any one of Claims 1 to 4, wherein the content of component (B) is from 0.01 to 5% by mass based on the total amount of the composition.

6. The cosmetic, drug or food according to any one of Claims 1 to 5, wherein the content of component (C) is 10% by mass or more based on the total amount of the composition.

7. The cosmetic, drug or food according to any one of Claims 1 to 6, further comprising a polyol.

8. The cosmetic, drug or food according to any one of Claims 1 to 7, wherein the aqueous composition is in a form of an aqueous solution or an emulsion having an aqueous phase as a continuous phase.

9. The cosmetic, drug or food according to any of Claims 1 to 8, further comprising as component (D) a plant extract being at least one selected from the group consisting of extracts from plants belonging to the family Brassicaceae, Poaceae, Clusiaceae, Scrophulariaceae, Zingiberaceae, Caprifoliaceae, Rosaceae, Leguminosae, Rutaceae, Magnoliaceae, and Saxifragaceae and seaweeds.

10. The cosmetic, drug or food according to any of Claims 1 to 8, further comprising as component (D) at least one selected from anti-inflammatory agents, moisturizing agents, amino acids, whitening agents, singlet oxygen quenchers, antioxidants, blood circulation accelerators, sebum secretion inhibitors, antibacterial agents, and keratolytic agents.

11. The cosmetic, drug or food according to any of Claims 1 to 8, further comprising as component (D) at least one skin-activating component selected from the group consisting of γ-amino-β-hydroxybutyric acid or salts thereof, glycyrrhizic acid or salts thereof, γ-aminobutyric acid or salts thereof, L-carnitine or salts thereof, rhododendrol, N-methyl-L-serine, niacinamide, ascorbic acid-2-glucoside, and isopropyl methylphenol.

12. The cosmetic, drug or food according to any one of Claims 1 to 11, the aqueous composition being a composition for external use on skin.

13. The cosmetic, drug or food according to any one of claims 1 to 12, wherein the aqueous composition has a viscosity at 25°C of 100 mPa·s or less, measured with a B-type viscometer.

14. Use of an agent for inhibiting water evaporation of a cosmetic, drug or food comprising an aqueous composition, wherein the agent comprises the following components (A) and (B) as active ingredients:
(A) a polyoxyethylene alkyl or alkenyl ether having an alkyl or alkenyl group having 20 to 24 carbon atoms and an average molar number of ethylene oxide added of 1.5 to 4; and
(B) a water-soluble polymer.

15. A method for moisturizing skin, wherein the method comprises applying an aqueous composition to skin, wherein the aqueous compostion comprises the following components (A), (B), and (C):
(A) a polyoxyethylene alkyl or alkenyl ether having an alky or alkenyl group having 20 to 24 carbon atoms and an average molar number of ethylene oxide added of 1.5 to 4;
(B) a water-soluble polymer; and
(C) water.

## Patentansprüche

1. Kosmetik, Arzneimittel oder Nahrungsmittel, enthalten in einem Behälter, umfassend eine wässrige Zusammensetzung, die die folgenden Komponenten (A), (B) und (C) enthält:
(A) einen Polyoxyethylenalkyl- oder -alkenylether mit einer Alkyl- oder Alkenylgruppe mit 20 bis 24 Kohlenstoffatomen und einer durchschnittlichen molaren Zahl von zugegebenem Ethylenoxid von 1,5 bis 4,
(B) ein wasserlösliches Polymer und
(C) Wasser.

2. Kosmetik, Arzneimittel oder Nahrungsmittel gemäß Anspruch 1, worin die Komponente (A) ein Polyoxyethylenalkyl- oder -alkenylether mit einer Alkyl- oder Alkenylgruppe mit 20 bis 24 Kohlenstoffatomen und einer durchschnittlichen molaren Zahl von zugegebenem Ethylenoxid von 1,5 bis 2,5 ist.

3. Kosmetik, Arzneimittel oder Nahrungsmittel gemäß Anspruch 1 oder 2, worin der Gehalt der Komponente (A) von 0,05 bis 20 Masse-% ist, bezogen auf die Gesamtmenge der Zusammensetzung.

4. Kosmetik, Arzneimittel oder Nahrungsmittel gemäß einem der Ansprüche 1 bis 3, worin die Komponente (B) zumindest eine ist, ausgewählt aus der Gruppe, bestehend aus Carboxyvinylpolymeren, Alkylacrylat/Methacrylat-Copolymeren, Xanthangummi, Hydroxypropylmethylcellulose, Polyacrylamiden und (Natriumacrylat/Natriumacryloyldimethyltaurat)-Copolymeren.

5. Kosmetik, Arzneimittel oder Nahrungsmittel gemäß einem der Ansprüche 1 bis 4, worin der Gehalt der Komponente (B) von 0,01 bis 5 Masse-%, bezogen auf die Gesamtmenge der Zusammensetzung, ist.

6. Kosmetik, Arzneimittel oder Nahrungsmittel gemäß einem der Ansprüche 1 bis 5, worin der Gehalt der Komponente (C) 10 Masse-% oder mehr ist, bezogen auf die Gesamtmenge der Zusammensetzung.

7. Kosmetik, Arzneimittel oder Nahrungsmittel gemäß einem der Ansprüche 1 bis 6, weiterhin enthaltend ein Polyol.

8. Kosmetik, Arzneimittel oder Nahrungsmittel gemäß einem der Ansprüche 1 bis 7, worin die wässrige Zusammensetzung in einer Form einer wässrigen Lösung oder einer Emulsion mit einer wässrigen Phase als kontinuierliche Phase ist.

9. Kosmetik, Arzneimittel oder Nahrungsmittel gemäß einem der Ansprüche 1 bis 8, weiterhin enthaltend als Komponente (D) einen Pflanzenextrakt, der zumindest einer ist, ausgewählt aus der Gruppe, bestehend aus Extrakten von Pflanzen, die zur Familie Brassicaceae, Poaceae, Clusiaceae, Scrophulariaceae, Zingiberaceae, Caprifoliaceae, Rosaceae, Leguminosae, Rutaceae, Magnoliaceae und Saxifragaceae und Algen.

10. Kosmetik, Arzneimittel oder Nahrungsmittel gemäß einem der Ansprüche 1 bis 8, weiterhin enthaltend als Komponente (D) zumindest eine, ausgewählt aus entzündungshemmenden Mitteln, Feuchtigkeitsmitteln, Aminosäuren, Weißmachern, Singulettsauerstoff-Abschreckmittel, Antioxidantien, Blutzirkulationsbeschleudiger, Sebumsekretionsinhibitoren, antibakteriellen Mitteln und keratolytischen Mitteln.

11. Kosmetik, Arzneimittel oder Nahrungsmittel gemäß einem der Ansprüche 1 bis 8, weiterhin enthaltend als Komponente (D) zumindest eine hautaktivierende Komponente, ausgewählt aus der Gruppe, bestehend aus γ-Amino-β-hydroxybuttersäure oder Salzen davon, Glycyrrhicinsäure oder Salzen davon, γ-Aminobuttersäure oder Salzen davon, L-Carnitin oder Salzen davon, Rhododendrol, N-Methyl-L-serin, Niacinamid, Ascorbinsäure-2-glucosid und Isopropylmethylphenol

12. Kosmetik, Arzneimittel oder Nahrungsmittel gemäß einem der Ansprüche 1 bis 11, wobei die wässrige Lösung eine Zusammensetzung für externe Verwendung auf der Haut ist.

13. Kosmetik, Arzneimittel oder Nahrungsmittel gemäß einem der Ansprüche 1 bis 12, worin die wässrige Zusammensetzung eine Viskosität bei 25°C von 100 mPa·s oder weniger hat, gemessen mit einem Viskometer vom B-Typ.

14. Verwendung eines Mittels zur Inhibition zur Wasserverdampfung eines Kosmetikums, Arzneimittel oder Nahrungsmittels, umfassend eine wässrige Zusammensetzung, worin das Mittel die folgenden Komponenten (A) und (B) als aktive Bestandteile enthält:
(A) ein Polyoxyethylenalkyl- oder -alkenylether mit einer Alkyl- oder Alkenylgruppe mit 20 bis 24 Kohlenstoffatomen und einer durchschnittlichen molaren Zahl von zugegebenem Ethylenoxid von 1,5 bis 4,
(B) ein wasserlösliches Polymer.

15. Verfahren zum Befeuchten von Haut, worin das Verfahren die Auftragung einer wässrigen Zusammensetzung auf die Haut enthält, worin die wässrige Zusammensetzung die folgenden Komponenten (A), (B) bis (C) enthält:
(A) einen Polyoxyethylenalkyl- oder -alkenylether mit einer Alkyl- oder Alkenylgruppe mit 20 bis 24 Kohlenstoffatomen und einer durchschnittlichen molaren Zahl von zugegebenem Ethylenoxid von 1,5 bis 4,
(B) ein wasserlösliches Polymer und
(C) Wasser.

## Revendications

1. Cosmétique, médicament ou aliment contenu dans un récipient, comprenant une composition aqueuse comprenant les composants suivants (A), (B), et (C) :
(A) un éther d'alkyle ou d'alcényle de polyoxyéthylène ayant un groupe alkyle ou alcényle ayant 20 à 24 atomes de carbone et un nombre molaire moyen d'oxyde d'éthylène ajouté de 1,5 à 4 ;
(B) un polymère hydrosoluble ; et
(C) de l'eau.

2. Cosmétique, médicament ou aliment selon la revendication 1, dans lequel le composant (A) est un éther d'alkyle ou d'alcényle de polyoxyéthylène ayant un groupe alkyle ou alcényle ayant 20 à 24 atomes de carbone et un nombre molaire moyen d'oxyde d'éthylène ajouté de 1,5 à 2,5.

3. Cosmétique, médicament ou aliment selon la revendication 1 ou 2, dans lequel la teneur du composant (A) est de 0,05 à 20 % en masse sur la base de la quantité totale de la composition.

4. Cosmétique, médicament ou aliment selon l'une quelconque des revendications 1 à 3, dans lequel le composant (B) est au moins un sélectionné dans le groupe constitué de polymères carboxyvinyliques, de copolymères d'acrylate/méthacrylate d'alkyle, de la gomme de xanthane, de l'hydroxypropylméthylcellulose, de polyacrylamides, et de copolymères (acrylate de sodium / acryloyldimethyltaurate de sodium).

5. Cosmétique, médicament ou aliment selon l'une quelconque des revendications 1 à 4, dans lequel la teneur du composant (B) est de 0,01 à 5 % en masse sur la base de la quantité totale de la composition.

6. Cosmétique, médicament ou aliment selon l'une quelconque des revendications 1 à 5, dans lequel la teneur du composé (C) est de 10 % en masse ou plus sur la base de la quantité totale de la composition.

7. Cosmétique, médicament ou aliment selon l'une quelconque des revendications 1 à 6, comprenant en outre un polyol.

8. Cosmétique, médicament ou aliment selon l'une quelconque des revendications 1 à 7, dans lequel la composition aqueuse est sous une forme d'une solution aqueuse ou d'une émulsion ayant une phase aqueuse en tant que phase continue.

9. Cosmétique, médicament ou aliment selon l'une quelconque des revendications 1 à 8, comprenant en outre comme composant (D) un extrait de plante qui est au moins une sélectionné dans le groupe constitué d'extraits de plantes appartenant à la famille des Brassicaceae, des Poaceae, des Clusiaceae, des Scrophulariceae, des Zingiberaceae, des Caprifoliaceae, des Rosaceae, des Leguminosae, des Rutaceae, des Magnoliaceae, et des Saxifragaceae et des algues.

10. Cosmétique, médicament ou aliment selon l'une quelconque des revendications 1 à 8, comprenant en outre comme composant (D) au moins un sélectionné parmi des agents anti-inflammatoires, des agents hydratants, des acides aminés, des agents de blanchiment, des agents d'extinction d'oxygène singulet, des antioxydants, des accélérateurs de circulation sanguine, des inhibiteurs de sécrétion de sébum, des agents antibactériens, et des agents kératolytiques.

11. Cosmétique, médicament ou aliment selon l'une quelconque des revendications 1 à 8, comprenant en outre comme composant (D) au moins un composant activant la peau sélectionné dans le groupe constitué de l'acide γ-amino-β-hydroxybutyrique ou de sels de celui-ci, de l'acide glycyrrhizique ou de sels de celui-ci, de l'acide γ-aminobutyrique ou de sels de celui-ci, de la L-carnitine ou de sels de celle-ci, du rhododendrol, de la N-méthyl-L-sérine, de la niacinamide, de l'acide ascorbique-2-glucoside et de l'isopropylméthylphénol.

12. Cosmétique, médicament ou aliment selon l'une quelconque des revendications 1 à 11, la composition aqueuse étant une composition pour un usage externe sur la peau.

13. Cosmétique, médicament ou aliment selon l'une quelconque des revendications 1 à 12, dans lequel la composition aqueuse a une viscosité à 25 °C de 100 mPa.s ou moins, mesurée avec un viscosimètre de type B.

14. Utilisation d'un agent pour inhiber l'évaporation d'eau d'un cosmétique, d'un médicament ou d'un aliment comprenant une composition aqueuse, dans laquelle l'agent comprend les composants (A) et (B) suivants comme ingrédients actifs :
(A) un éther d'alkyle ou d'alcényle de polyoxyéthylène ayant un groupe alkyle ou alcényle ayant 20 à 24 atomes de carbone et un nombre molaire moyen d'oxyde d'éthylène ajouté de 1,5 à 4 ; et
(B) un polymère hydrosoluble.

15. Méthode d'hydratation de la peau, dans laquelle la méthode comprend l'application d'une composition aqueuse sur la peau, dans laquelle la composition aqueuse comprend les composants (A), (B), et (C) suivants :
(A) un éther d'alkyle ou d'alcényle de polyoxyéthylène ayant un groupe alkyle ou alcényle ayant 20 à 24 atomes de carbone et un nombre molaire moyen d'oxyde d'éthylène ajouté de 1,5 à 4 ;
(B) un polymère hydrosoluble ; et
(C) de l'eau.
